# EUROPEAN PATENT SPECIFICATION

(11) **EP 0 661 999 B1**
(45) Date of publication and mention of the grant of the patent: **30.12.1998**
(21) Application number: 93920971.4
(22) Date of filing: 20.09.1993
(51) Int. Cl.: A61K 39/21, C07K 7/02, G01N 33/569, C12N 15/49

(54) **ANTI-FELINE IMMUNODEFICIENCY VIRUS (FIV) VACCINES**
VAKZINE GEGEN IMMUNODEFIZIENZ-VIRUS DER KATZE (KIV)
VACCINS DESTINES A LUTTER CONTRE LE VIRUS IMMUNODEFICITAIRE FELIN

(30) Priority: 21.09.1992 GB 9219936
(43) Date of publication of application: 12.07.1995
(73) Proprietor: Mallinckrodt Veterinary, Inc., Mundelein Illinois 60060 (US)
(72) Inventor: FRANCIS, Michael, James Pitman-Moore Ltd., Uxbridge Middlesex UB9 6LS (GB)
(74) Representative: Dzieglewska, Hanna Eva
(86) International application number: GB9301974
(87) International publication number: WO9406471

(56) References cited:
- WO-A-92/09632
- WO-A-92/15684
- WO-A-92/22573
- WO-A-93/01304

## Description

The present invention relates to synthetic feline immuno-deficiency virus (FIV) peptides, their preparation and their use as vaccines and diagnostic reagents.

FIV is a recently discovered T-lymphotropic lentivirus which infects cats to produce an AIDS-like syndrome. FIV, while exhibiting morphological and pathological similarity, has however been shown to be antigenically distinct from the human immunodeficiency virus (HIV) (Pederson et al., Science 235: 790-793, 1987). Infected cats and kittens show a generally debilitating AIDS-like disease with intermittent symptoms (eg. lymphadenopathy, leucopenia and anaemia), characterised by a severe impairment of immune function as a result of loss of CD⁴⁺ T cells, resulting in susceptibility to secondary opportunistic infection and leading ultimately to death.

Epidemiological studies have shown FIV infection to be widespread worldwide and the disease is rapidly acquiring significant clinical importance from a veterinary point of view. Efforts have accordingly recently begun to be directed to the development of vaccines against FIV but whilst preliminary results with vaccines based on whole infected cells or whole virus have proved promising, (Yamamoto et al., AIDS Research and human retroviruses 7: 911-922, 1991), there are as yet no reports in the literature of successful immunisation of cats against FIV infection using a sub-unit or peptide-based vaccine. No commercial vaccines are currently available.

Despite its ubiquitous presence, FIV does not appear to be capable of cat to human transmission. Nonetheless, FIV shares with HIV and other mammalian lentiviruses similarities in genome organisation, biological properties, the propensity for persistent infection in the natural host, with its concomitant pathological manifestations (eg. decline in CD4+ lymphocytes, both in vivo and in vitro, gradual loss of immune function, and opportunistic infection). Thus the development of experimental models of non-human lentivirus infections, such as FIV, may facilitate the design of vaccine and therapeutic strategies for HIV infection of humans.

The molecular structure of FIV, in terms of genome organisation and nucleotide sequence, has been studied (Talbot et al., PNAS, 86: 5743-5747, 1989; Olmstead et al., PNAS 86: 8088-8092, 1989) but as yet progress towards identifying regions of particular immunogenic importance has been slow and most proposals for vaccines currently under investigation involve whole undisrupted virus.

There is therefore a continuing need for an effective candidate vaccine against FIV, particularly for a sub-unit or peptide-based vaccine, both for combatting FIV infection in cats and kittens and to serve as a useful animal model for HIV infection in man in which experimental vaccines may be evaluated. The present invention is directed towards providing such a candidate vaccine.

A number of approaches to virus vaccine development are available. One approach, as mentioned above is to use whole undisrupted virus, either in inactivated or live attenuated form. Such an approach, although generally effective and in the case of FIV initially promising, is not always problem-free as on occasion the virus may be incompletely inactivated or insufficiently attenuated and may infect rather than protect the immunised host. In the case of an infection as serious as FIV, this is a risk which cannot be taken lightly and a synthetic vaccine which would eliminate all aspects of virus handling in production and vaccination would represent a more attractive alternative.

With this aim in mind efforts have been directed during the last decade to the development of vaccines based on synthetic peptides, containing antigenic sites of importance in raising protective immunity (Francis, Sci. Progress 74: 115-130, 1990). Such peptides may mimic important continuous or discontinuous epitopes (a discontinuous epitope is one comprising amino acid residues from different proteins or from different regions of the same protein). The first demonstration that peptides could elicit protective immunity in vivo in addition to neutralising activity in vitro was obtained in 1982 using a peptide from the VP1 coat protein of foot-and-mouth disease virus (FDMV) in a guinea-pig animal model. This has subsequently been supported by the demonstration of protective immunity in cattle and pigs. Although no commercial product has yet reached the market place, peptides have now been used to elicit immune responses against a wide variety of viruses, including hepatitis B virus, influenza virus, herpes simplex, human rotavirus, HIV, bovine rotavirus, bovine enterovirus and many others.

In the search for a peptide-based vaccine against FIV, we have directed our efforts towards the envelope protein of FIV which we believe may be important for virus infectivity and a major target, both for cytotoxic T cells and neutralising antibodies in the host immune response to FIV infection.

In co-pending International Patent Application No. PCT/EP93/01860 filed on 15 July 1993 (WO-A-9402612), we proposed a FIV vaccine based on synthetic peptides from the region spanning residues 483 to 567 of the FIV envelope protein.

WO92/09632 describes peptide fragments of the env protein of FIV which are recognized by antibodies in the serum of FIV-infected animals and which when used for immunization raise antibodies which recognize the immunogenic peptides, the FIV env protein and FIV.

We now propose, according to the present invention, a further series of FIV envelope protein peptides as suitable candidates for FIV vaccines or diagnostic agents.

In one aspect the present invention thus provides a synthetic polypeptide comprising an amino acid sequence selected from:

| FIV envelope protein | | |
|---|---|---|
| | Residue Numbers | Sequence |
| SEQ ID. NO. | | |
| 1 | 377-417 | CQRTQSQPGLWLRAISSWKQRNRWEWRPDFKSKKMKISLQC; and/or |
| 2 | 462-490 | CRWNVGDNTSLIDTCGETQNVSGANPVDC; and/or |
| 3 | 354-377 | CGWWNQIAYYNSCRWESTDVKFHC; and/or |
| 5 | 417-445 | CNSTQNLTFAMRSSGDYGEVTGAWIEFGC; and/or |
| 6 | 320-339 | CTDPLQIPLINYTFGPNQTC; and/or |
| 7 | 161-190 | LFIGVGIYLGTAKAQVVWRLPPLVVPVEES; and/or |
| 8 | 248-267 | CRRGRIWKRWNETITGPSGC; and/or |
| 10 | 716-736 | NMTINQTIWNHGNITLGEWYN; |

or an antigenic fragment, functionally-equivalent variant retaining immunogenic activity or pharmaceutically acceptable salt thereof.

A further aspect of the invention provides such synthetic polypeptides and antigenic fragments, functionally-equivalent variants and pharmaceutically acceptable salts thereof for use in combatting FIV infection in animals e.g. by stimulating an immune response against FIV.

Alternatively viewed, the invention can also be seen to provide the use of such synthetic polypeptides, and antigenic fragments, functionally equivalent variants and pharmaceutically acceptable salts thereof according to the present invention, in the preparation of a composition for combatting FIV infection in animals, preferably for stimulating an immune response against FIV.

The term "polypeptide" as used herein defines both long chain polypeptides and shorter peptide sequences.

As mentioned above, included within the scope of invention are functionally-equivalent variants and fragments of synthetic FIV polypeptides according to the invention. "Functionally equivalent" as used above in relation to the polypeptide amino acid sequences defines polypeptides related to or derived from the above native FIV envelope protein polypeptide sequences where the amino acid sequence has been modified by single or multiple amino acid substitution, addition or deletion, and also sequences where the amino acids have been chemically modified, including by glycosylation or deglycosylation, but which nonetheless retain immunogenic or other FIV-combatting activity eg. are capable of generating a host-protective immune response for example by raising neutralising antibodies and/or functional immunity in the host. Such functionally-equivalent variants may occur as natural biological variations or may be prepared using known techniques, for example functionally equivalent recombinant polypeptides may be prepared using the known techniques of site-directed mutagenesis, random mutagenesis, or enzymatic cleavage and/or ligation of amino acids.

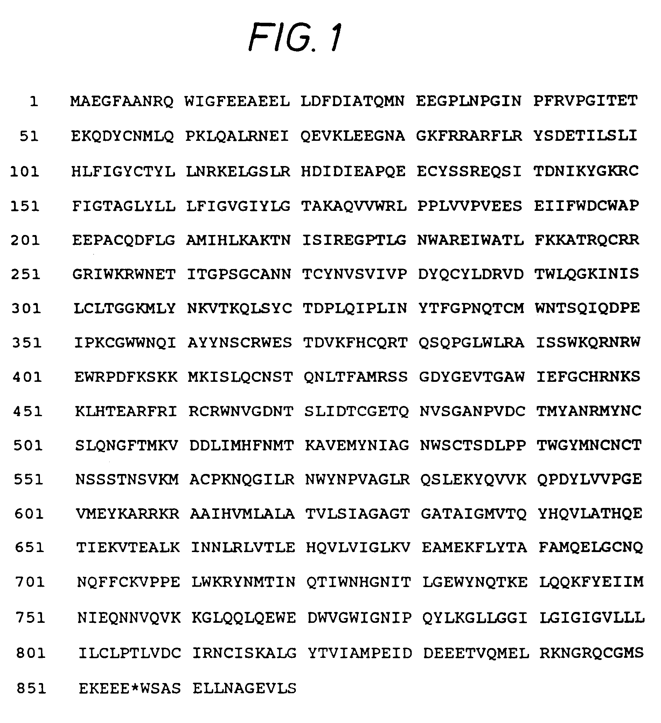

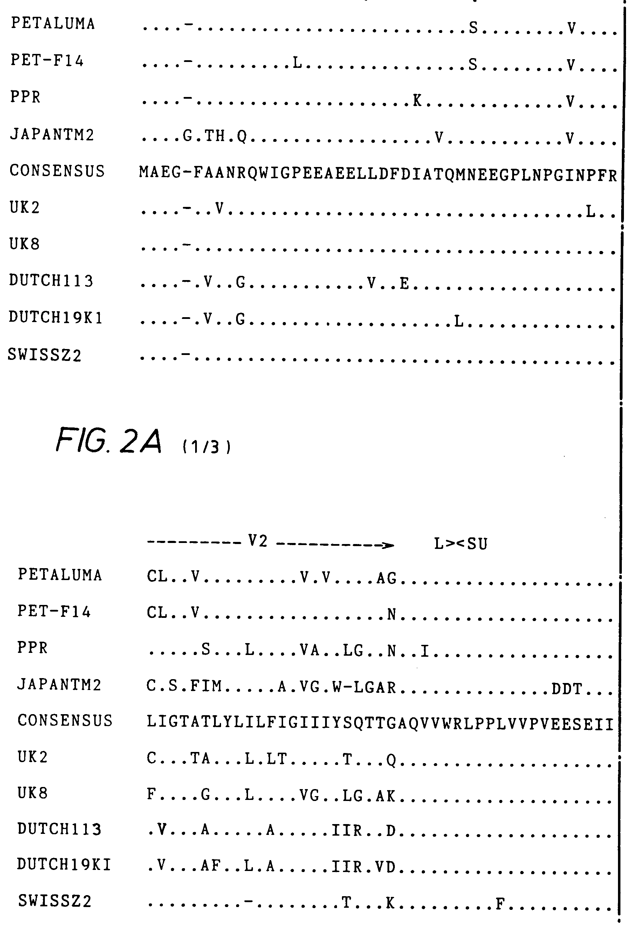

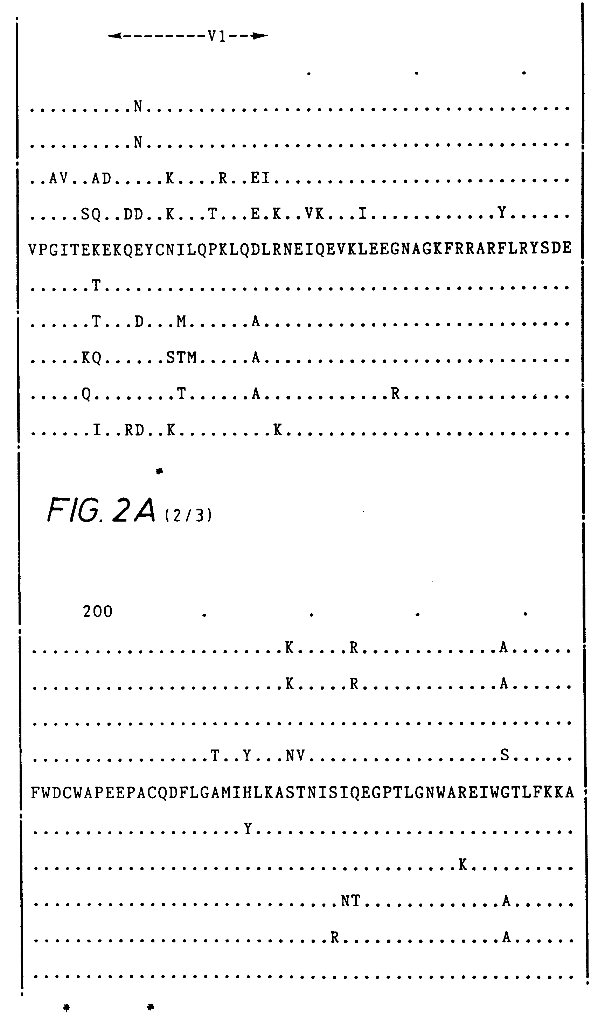

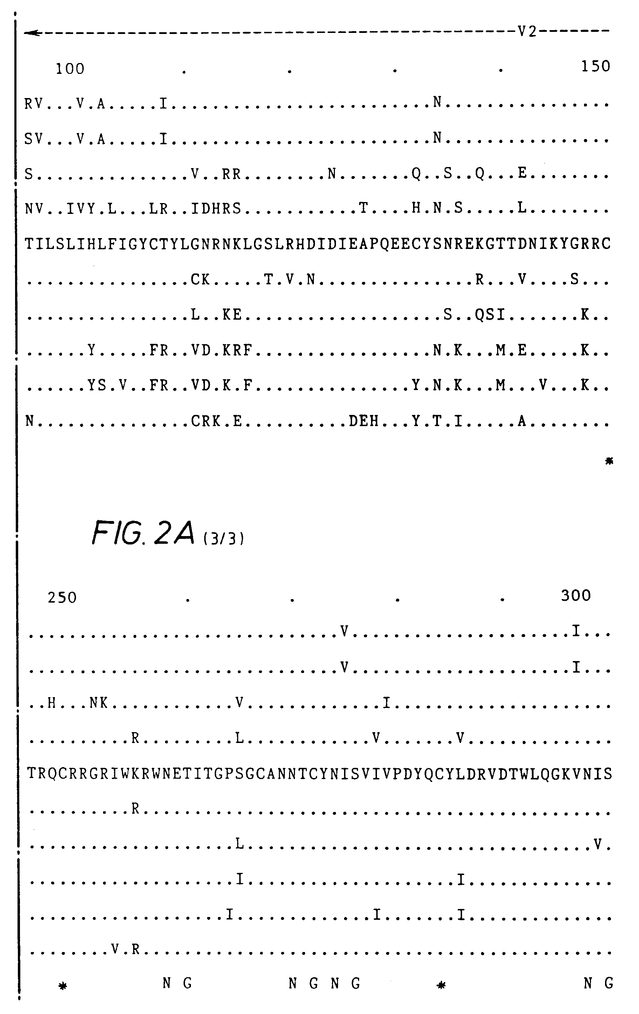

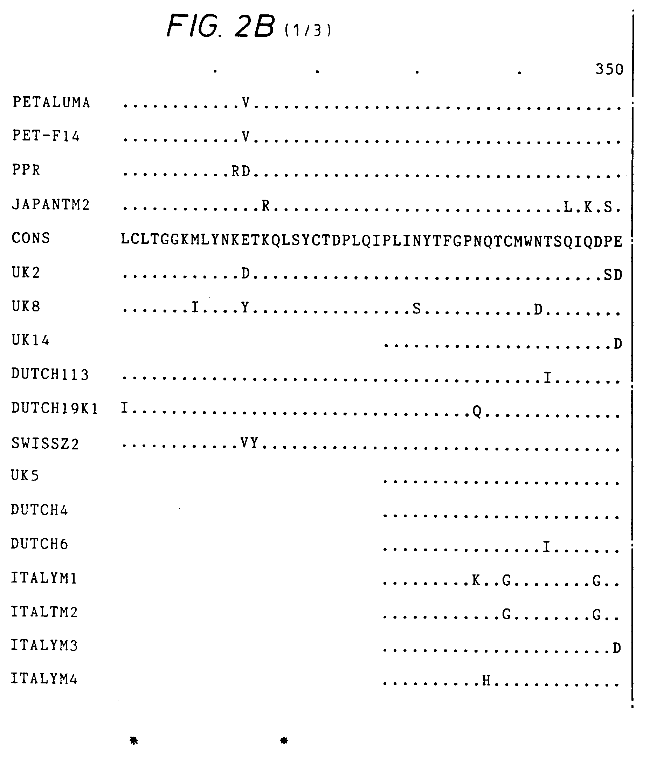

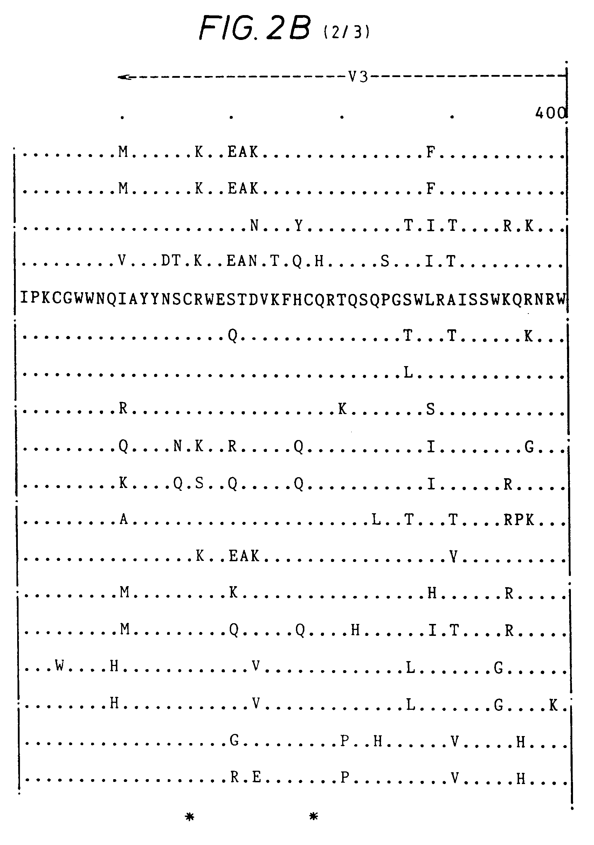

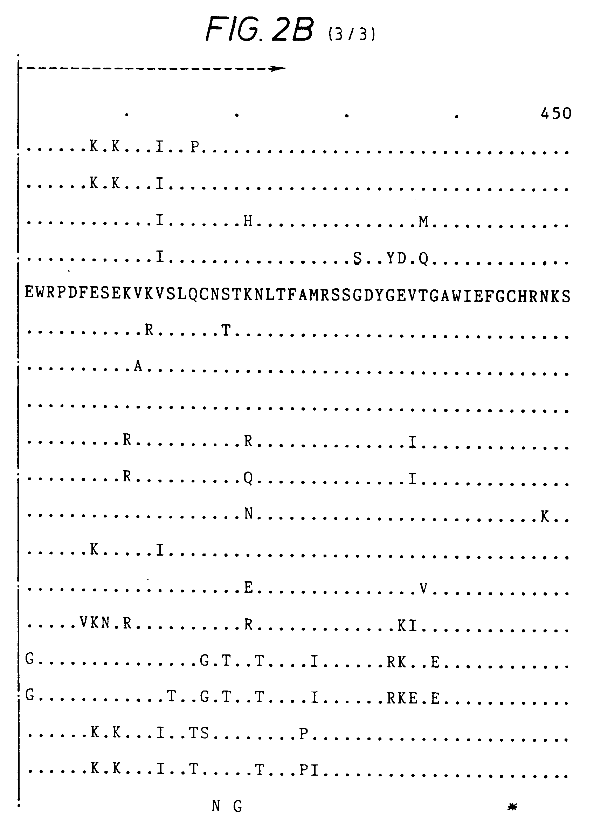

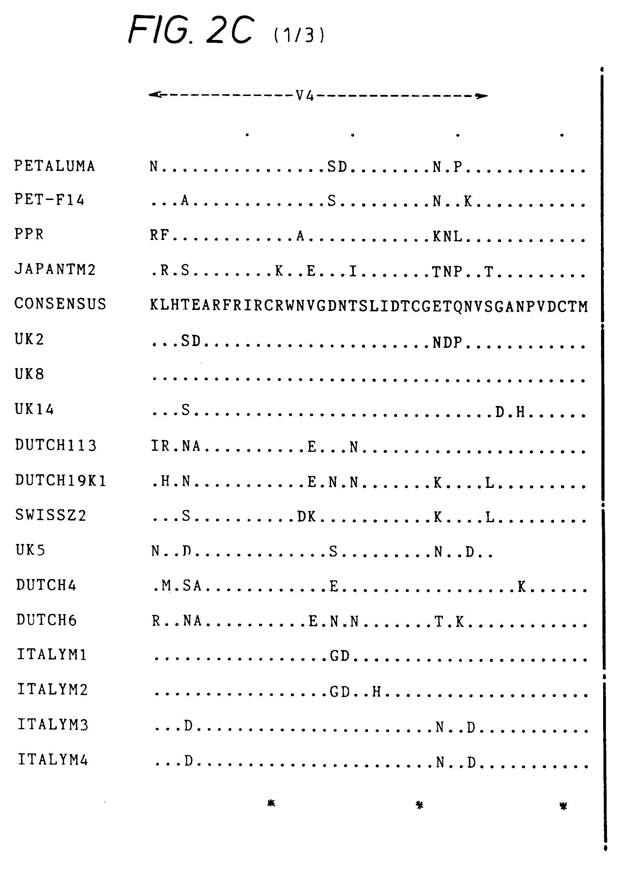

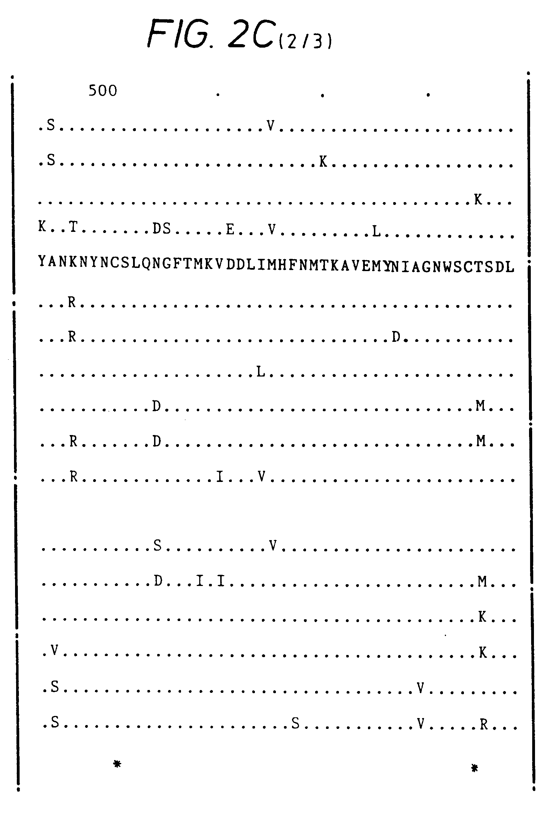

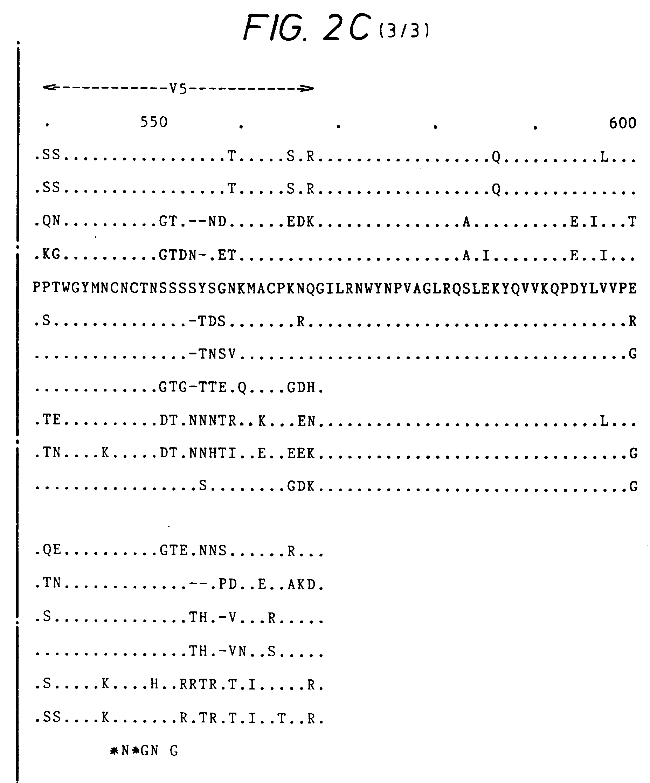

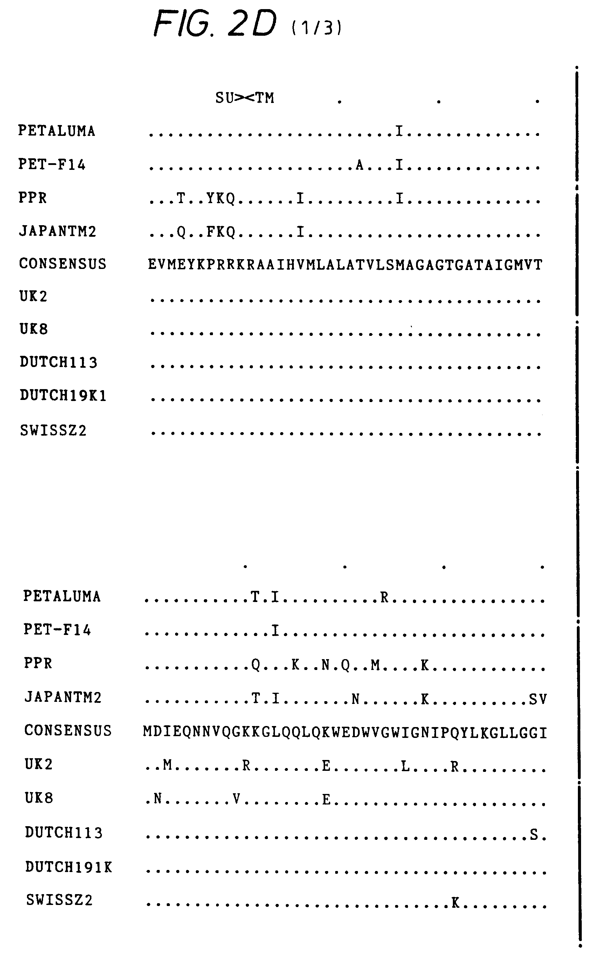

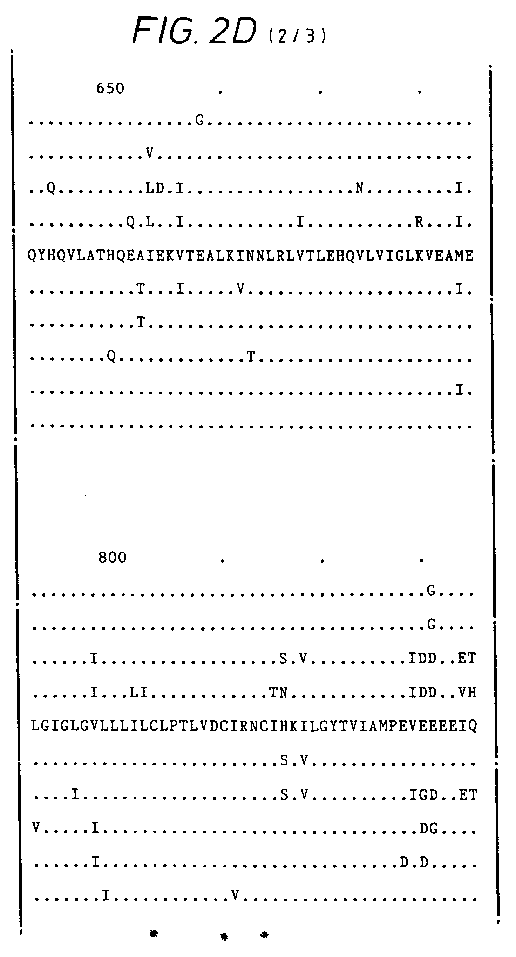

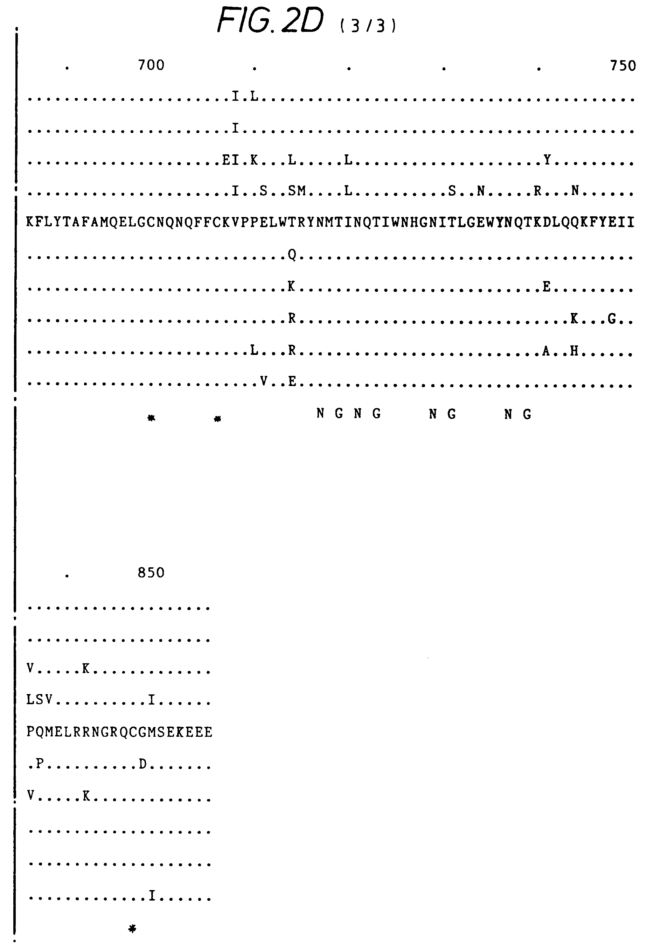

Thus for example the above-mentioned amino acid sequences were determined principally from a combination of the sequences of the FIV UK 8 and Petaluma isolates. Figure 1 shows a composite of the amino acid sequences of the envelope proteins of the FIV UK 8 and Petaluma isolates, from which the synthetic polypeptides of the present invention were derived. Variations in the sequence may however occur between different strains or serotypes, isolates of different geographical origin or even between different isolates from the same host. Different FIV isolates and sequence divergence between them are described for example by Rigby et al., in Journal of General Virology (1993), 74, 425-436. A comparison and alignment of the predicted amino acid sequences of the envelope protein of various representative FIV isolates is presented in Figure 2. The asterisk (*) denotes conserved cysteine residues. NG, conserved potential N-linked glycosylation; L><SU, putative cleavage site of hydrophobic leader; SU><TM, putative cleavage site between surface glycoprotein and transmembrane protein. Such variants are included within the scope of this invention.

As mentioned above, modification of the amino acid sequences to obtain functionally-equivalent variant sequences may be by amino acid substitution, as long as the immunogenicity of the polypeptide is not affected. Thus for example, an amino acid may be replaced by another which preserves the physicochemical character of the polypeptide or its epitope(s) eg. in terms of charge density, hydrophilicity/hydrophobicity, size and configuration and hence preserve the immunological structure. For example A may be replaced by G or vice versa, V by A, L or G; K by R; S by T or vice versa; E by D or vice versa; and Q by N or vice versa.

Generally, the substituting amino acid has similar properties eg. hydrophobicity, hydrophilicity, electronegativity, bulky side chains etc. to the amino acid being replaced.

"Addition" variants include amino and/or carboxyl terminal fusions, for example by addition of amino acid sequences of up to 300 eg. up to 200 or 100 residues, as well as intrasequence insertions of single or multiple amino acids. Amino acid sequences added may be those provided in the corresponding positions in the FIV envelope protein or other amino acids, eg. the whole or parts of other polypeptides or proteins. Longer peptides may comprise multiple copies of one or more of the polypeptide sequences. Alternatively, multiple copies of the polypeptides (e.g. 5 to 20, preferably 8 to 15) may be coupled to a polyamino acid backbone, eg. a polylysine backbone to form so-called multiple antigen peptides (MAPs) as described for example by Tam in PNAS 85: 5400-5413, 1988).

Insertional amino acid sequence variants are those in which one or more amino acid residues are introduced into a predetermined site in the protein although random insertion is also possible with suitable screening of the resulting product. Deletional variants are characterised by the removal of one or more amino acids from the sequence. Preferably, deletions or insertions are made in adjacent pairs eg. a deletion of two residues or insertion of two molecules. In all cases the proviso is that the modification preserves the immunogenicity of the polypeptide.

Exemplary functionally-equivalent variant polypeptides may thus include those displaying at least 50%, eg. at least 60 or 70%, or more preferably greater than 80% amino acid sequence homology. It should be noted however that functionally-equivalent variants, may exhibit overall sequence homology below the given percentages, but still fall within the scope of the present invention where they have conserved regions of homology.

It may in certain cases be convenient to include, where one does not occur naturally, one or more cysteine residues at the termini of the polypeptides, for example to enable specific carrier linkage or to permit disulphide bonding - this may be desirable to enable the polypeptides to mimic antigenic loops such as may appear on the surface of proteins and thereby enhance their immunogenicity. It will be noted that polypeptides 1 to 6 and 8 mentioned above, have N- and C-terminal cysteine residues.

A fatty acid or hydrophobic tail may also be added to the peptides to enhance immunogenicity and facilitate incorporation into delivery vehicles such as liposomes, novosomes and ISCOMS (Reid, Vaccine 10: 597-601, 1992).

The amino acid residues of the synthetic polypeptides of the invention may be chemically modified, particularly at the ends of the molecule, and may take the form, for example, of amino acid esters or amides. Thus, for example, N- or C-terminal residues, eg. N- or C-terminal, particularly C-terminal cysteine residues may be chemically blocked or protected for example by an acetamido or other protecting group. A wide range of blocking groups are known in the art and may be used, including for example sulphonate, carboxymethyl, carboxamidomethyl, amino ethyl and similar groups.

The polypeptide may be linked to a carrier in order to create a conjugate which is immunogenically active. Any appropriate physiologically acceptable carrier may be employed, for example, a protein such as bovine serum albumin, thyroglobulin, ovalbumin or keyhole limpet hemocyanin. Recently, the concept of presenting multiple copies of viral peptides on the surface of particulate structures in a manner that resembles a virion has received much attention. This can be achieved either by producing virus/peptide chimaeras, eg. with poliovirus, or by linking the peptide to proteins which naturally self assemble into particles such as hepatitis B surface antigen, the Ty protein from yeast, or the core protein from hepatitis virus (HBcAg). The polypeptide may also be linked to other FIV proteins or polypeptides thereby providing both an immunogen and a multivalent vaccine at the same time.

Rather than link a carrier sequence to a peptide in this way, a polypeptide may be prepared which itself incorporates an appropriate carrier sequence ie. as a fusion protein comprising the synthetic polypeptide(s) of the invention or a longer sequence incorporating such a polypeptide linked to a heterologous carrier sequence, as will be described in more detail below. Such carrier proteins are generally chosen so that the resultant product is physiologically acceptable.

Other modifications which may be made to enhance immunogenicity of the synthetic polypeptides include the introduction of, or coupling to longer amino acid sequences containing FIV helper T-cell epitopes. Particular mention may be made in this regard of particulate carrier proteins such as HBcAg which contain helper T-cell epitopes and thus have the dual advantages of a highly immunogenic mode of antigen presentation and the presence of helper T-cell epitopes. Such modifications, and many others which may enhance antigen presentation and/or delivery to the immune system, including those described above are well known in the art and are discussed and reviewed by Francis in Vaccines, Eds. Gregoriadis et al, Plenum Press, New York, p 13-23, 1991; Sci. Progress 74: 115-130, 1990 and by Francis & Clarke in Meth. Enzymol., 178: 659-676, 1989.

The synthetic polypeptides of the invention may be presented as pharmaceutically or physiologically acceptable salts eg. acid addition salts. This may include both organic and inorganic salts such as those prepared for example from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzene-sulfonic, naphthalenesulfonic, propionic, and the like. Preferably, the acid addition salts are those prepared from hydrochloric acid, acetic acid, or succinic acid. Such salts may be prepared by conventional methods well known to those skilled in the art.

Alternatively the peptide may be converted into a carboxylic acid salt, such as an ammonium or alkali metal salt eg. a sodium, potassium, or lithium salt etc.

As mentioned above, the synthetic FIV polypeptides according to the invention may be used to combat FIV infection in animals, and preferably to stimulate a host immune response against FIV. Such an immune response may comprise elements of both humoral and/or cell-medicated immunity to protect the host from FIV infection and/or kill or inhibit the virus, and may thus for example include the generation of immune effector molecules, antibodies or cells which damage, inhibit or kill the virus. Commonly, such a host-protective immune response may be manifested by the generation of antibodies which are able to neutralise the virus.

Thus, one of the ways in which the synthetic FIV polypeptides of the invention may exert their host protective effects is by raising neutralising antibodies which inhibit the growth and/or maintenance of the virus. Such neutralising antibodies, which may be mono-or polyclonal, form a further aspect of the invention as do vaccine compositions containing them and their use in the preparation of vaccine compositions for passively immunising hosts against FIV infection. Techniques for obtaining mono- or polyclonal antibodies are well known in the art.

FIV polypeptides according to the invention conveniently may be prepared by recombinant DNA technology using standard techniques such as those described for example by Sambrook et al., 1989, (Molecular Cloning, a Laboratory Manual, 2nd Edition, Cold Spring Harbour Press).

A further aspect of the present invention thus provides a nucleic acid molecule comprising a nucleotide sequence comprising a region of the FIV env gene encoding residues of the FIV envelope protein selected from:

### Residues

(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a portion thereof encoding an antigenic fragment of FIV, or a sequence which is degenerate or has greater than 60% sequence homology therewith or which hybridises under conditions of high stringency with any such aforesaid sequence, for use in combatting FIV, preferably a vaccine against FIV infection in animals ie. a vaccine composition for stimulating an immune response against FIV.

Alternatively viewed, the invention also provides use of such a nucleic acid molecule in the preparation of a composition for combatting FIV, preferably a vaccine against FIV infection in animals.

A further aspect of the invention provides a nucleic acid molecule comprising a first nucleotide sequence corresponding to a region of the FIV env gene encoding residues of the FIV envelope protein selected from:

### Residues

(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a portion thereof encoding an antigenic fragment of FIV, or a sequence which is degenerate or has greater than 60% sequence homology therewith or which hybridises under conditions of high stringency with any such aforesaid sequence, together with at least one additional nucleotide sequence flanking said first nucleotide sequence, the additional nucleotide sequence comprising no more than 900 bases, preferably no more than 600 eg. no more than 300 bases.

Nucleic acid molecules according to the invention may be single or double stranded DNA, cDNA or RNA, and conveniently take the form of a recombinant nucleic acid molecule, preferably a recombinant DNA molecule.

The additional flanking sequences in the nucleic acid molecule according to the invention may be derived from the FIV env gene itself, from other regions of FIV DNA or from heterologous sources, and may be coding or non-coding. In a preferred aspect the flanking sequences may contain one or more restriction sites.

As mentioned above, variations in the env coding region may occur between different serotypes, isolates or strains of FIV, eg. strains of different geographical origin or even between different isolates from the same host and such variations in the region of the FIV env gene encoding the above-mentioned residues of the FIV envelope protein, which express as products capable of combatting FIV eg. by stimulating an immune response against FIV, are included in the scope of this aspect of the invention.

"Substantially homologous" as used herein includes those sequences having a sequence homology of approximately 60% or more, eg. 70% or 80% of more and wherein the nucleic acid encodes or is complementary to a sequence which encodes an FIV-combatting, eg. immunogenic polypeptide, and also functionally-equivalent allelic variants and related sequences modified by single or multiple base substitution, addition and/or deletion. By "functionally equivalent" is meant nucleotide sequences which encode immunoreactive or immunogenic polypeptides eg. polypeptides which are capable of eliciting antibodies eg. neutralising antibodies or functional immunity in the host, or which otherwise are capable of combatting the FIV virus.

The use of nucleotide sequences as defined above which hybridise with the above-mentioned regions of the FIV env gene, or with any degenerate, substantially homologous or functionally-equivalent sequence as defined above is also included within the scope of invention.

"Hybridisation" as used herein defines those sequences binding under non-stringent conditions (eg. 6 x SSC 50% formamide at room temperature) and washed under conditions of low stringency (eg. 2 x SSC, room temperature, more preferably 2 x SSC, 42°C) or conditions of higher stringency (eg. 2 x SSC, 65°C) (where SSC = 0.15M NaCl, 0.015M sodium citrate, pH 7.2). Generally speaking, sequences which hybridise under conditions of high stringency are included within the scope of the invention, as are sequences which, but for the degeneracy of the code, would hybridise under high stringency conditions.

Methods for producing such derivative related sequences, for example by site-directed mutagenesis, random mutagenesis, or enzymatic cleavage and/or ligation of nucleic acids are well known in the art, as are methods for determining whether the thus-modified nucleotide sequence has significant homology to the subject sequence, for example by hybridisation.

Provision of a nucleotide sequence according to the invention thus enables the synthetic FIV polypeptides to be obtained in significant quantities, thereby facilitating the development of anti-FIV vaccines and therapies.

In another aspect the present invention thus provides the use of a nucleic acid molecule comprising a nucleotide sequence encoding a polypeptide capable of raising neutralising antibodies against FIV which sequence incorporates one or more antigenic determinant-encoding regions from the regions of the FIV env gene encoding residues of the FIV envelope protein selected from:
(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a sequence which is degenerate, has greater than 60% sequence homology therewith or which hybridises under conditions of high stringency with any such aforesaid sequence, for the preparation of a composition for combatting FIV.

The nucleic acid molecules according to the invention as defined above can be expressed in appropriate expression systems well known in the art, to obtain recombinant synthetic FIV polypeptides of the invention.

The recombinant synthetic FIV polypeptides may thus be prepared by expression in a host cell containing a recombinant DNA molecule which comprises a nucleic acid molecule as broadly defined above, operatively linked to an expression control sequence, or a recombinant DNA cloning vehicle or vector containing such a recombinant DNA molecule. Alternatively, the polypeptides may be expressed by direct injection of a naked DNA molecule comprising a nucleotide sequence according to the invention into a host cell. Appropriate recombinant DNA technology and expression techniques are, as mentioned above, well described in the literature for example in Sambrook et al., 1989 (Supra).

The polypeptides so expressed may be fusion polypeptides comprising a FIV polypeptide according to the invention, and an additional polypeptide coded for by the DNA of the recombinant molecule fused thereto. This may for example be β-galactosidase, glutathione-S-transferase, yeast Ty particles, hepatitis core or surface antigen, transmembrane portions of membrane proteins or any other of the polypeptides commonly employed in fusion proteins in the art. Fusions with hepatitis core antigen are particularly preferred.

In an alternative form of fusion protein, one or more synthetic polypeptides of the invention may replace one or more antigenic epitopes, or parts thereof, of another protein such as hepatitis core antigen, yeast Ty particles or influenza virus haemagglutonin (HA).

Other aspects of the invention thus include cloning and expression vectors containing DNA comprising nucleotide sequences according to the invention. Such expression vectors include appropriate control sequences such as for example translational (eg. start and stop codes) and transcriptional control elements (eg. promoter-operator regions, ribosomal binding sites, termination stop sequences) linked in matching reading frame with the nucleic acid molecules of the invention.

The invention also includes transformed or transfected prokaryotic or eukaryotic host cells, or transgenic organisms containing a nucleotide sequence according to the invention as defined above, as well as methods for preparing a synthetic polypeptide of the invention by culturing host cells containing a nucleic acid molecule as defined above under conditions whereby said polypeptide is expressed and recovering said polypeptide thus produced.

Cloning or expression vectors according to the invention may include plasmids, phage and viruses, according to techniques well known in the art and may be expressed in a variety of different expression systems cells, including bacterial (eg. E. coli) yeast or mammalian expression systems. The polypeptides according to the invention may thus be expressed in genetically engineered cell lines eg. cell lines (such as BHK or Hela) transfected with, for example, a virus vector, and capable of constitutively expressing the FIV polypeptides according to the invention. Thus, for example, as typical of a suitable viral vector may be mentioned a recombinant vaccinia virus. As a convenient means of expression, a nucleic acid molecule according to this invention may be inserted into a plasmid vector downstream of a vaccinia virus promoter and flanked by vaccinia thymidine kinase (TK) sequences. The resultant recombinant vector is introduced into cells transfected with vaccinia virus. As a result of homologous recombination a TK⁻ recombinant vaccinia virus is generated which expresses the polypeptide.

Preferred expression systems according to the present invention include especially mammalian cell systems (eg. BHK cells) but also yeast, vaccinia, Baculovirus and bacterial systems such as E. coli or Salmonella.

The synthetic polypeptides may also be prepared by chemical synthesis, for example using solid phase synthesis, advantageously using a polypeptide synthesis apparatus, as commercially available. In such a synthesis, active side chain groupings (e.g. amino or carboxyl groups) of the respective amino acids will be protected and the final step will be deprotection and/or removal from the inert support to which the polypeptide is attached during synthesis. Solution-phase synthesis systems are also known in the art.

In building up the peptide chains, one can in principle start either at the C-terminal or the N-terminal although only the C-terminal starting procedure is in common use.

Thus, one can start at the C-terminal by reaction of a suitable derivative of, for example cysteine with a suitable protected derivative of glutamine. The cysteine derivative will have a free α-amino group while the other reactant will have either a free or activated carboxyl group and a protected amino group. After coupling, the intermediate may be purified for example by chromatography, and then selectively N-deprotected to permit addition of a further N-protected and free or activated amino acid residue. This procedure is continued until the required amino acid sequence is completed.

Carboxylic acid activating substituents which may, for example, be employed include symmetrical or mixed anhydrides, or activated esters such as for example p-nitrophenyl ester, 2,4,5,trichlorophenyl-ester, N-hydroxybenzotriazole ester (OBt), N-hydroxy-succinimidylester (OSu) or pentafluorophenylester (OPFP).

The coupling of free amino and carboxyl groups may, for example, be effected using dicyclohexylcarbodi-imide (DCC). Another coupling agent which may, for example, be employed is N-ethoxycarbonyl-2-ethoxy-1,2-dihydroquinoline (EEDQ).

In general it is convenient to effect the coupling reactions at low temperatures, for example, -20°C up to ambient temperature, conveniently in a suitable solvent system, for example, tetrahydro- furan, dioxan, dimethylformamide, methylene chloride or a mixture of these solvents.

It may be more convenient to carry out the synthesis on a solid phase resin support. Chloro-methylated polystyrene (cross-linked with 1% divinyl benzene) is one useful type of support; in this case the synthesis will start the C-terminal, for example by coupling N-protected cysteine to the support.

A number of suitable solid phase techniques are described by Eric Atherton, Christopher J. Logan, and Robert C. Sheppard, J. Chem. Soc. Perkin I, 538-46 (1981); James P. Tam, Foe S. Tjoeng, and R. B, Merrifield J. Am. Chem. Soc. 102, 6117-27 (1980); James P. Tam, Richard D. Dimarchi and R. B. Merrifield Int. J. Peptide Protein Res 16 412-25 (1980); Manfred Mutter and Dieter Bellof, Helvetica Chimica Acta 67 2009-16 (1984).

A wide choice of protecting groups for amino acids are known and are exemplified in Schröder, E., and Lübke, K., The Peptides, Vols. 1 and 2, Academic Press, New York and London, 1965 and 1966; Pettit, G.R., Synthetic Peptides, Vols. 1-4, Van Nostrand, Reinhold, New York 1970, 1971, 1975 and 1976; Houben-Weyl, Methoden der Organischen Chemie, Synthese von Peptiden, Band 15, Georg Thieme Verlag Stuttgart, NY, 1983; The Peptides, Analysis, synthesis, biology 1-7, Ed: Erhard Gross, Johannes Meienhofer, Academic Press, NY, San Fransisco, London; Solid phase peptide synthesis 2nd ed., John M. Stewaet, Janis D. Young, Pierce Chemical Company.

Thus, for example amine protecting groups which may be employed include protecting groups such as carbobenzoxy (Z-), t-butoxycarbonyl (Boc-), 4-methoxy-2,3,6-trimethyl-benzene sulphonyl (Mtr-), and 9-fluorenylmethoxycarbonyl (Fmoc-). It will be appreciated that when the peptide is built up from the C-terminal end, an amine protecting group will be present on the α-amino group of each new residue added and will need to be removed selectively prior to the next coupling step. One particularly useful group for such temporary amine protection is the Fmoc group which can be removed selectively by treatment with piperidine in an organic solvent.

Carboxyl protecting groups which may, for example be employed include readily cleaved ester groups such as benzyl (-OBZl), p-nitrobenzyl (-ONB), or t-butyl (-tOBu) as well as the coupling on solid supports, for example methyl groups linked to polystyrene.

It will be appreciated that a wide range of other such groups exists as, for example, detailed in the above-mentioned literature references, and the use of all such groups in the hereinbefore described processes fall within the scope of the present invention.

A wide range of procedures exists for removing amine-and carboxyl-protecting groups. These must, however, be consistent with the synthetic strategy employed. The side chain protecting groups must be stable to the conditions used to remove the temporary α-amino protecting groups prior to the next coupling step.

Amine protecting groups such as Boc and carboxyl protecting groups such as tOBu may be removed simultaneously by acid treatment, for example with trifluoro acetic acid.

A further aspect of the invention provides a process for the preparation of synthetic polypeptides according to the invention in which a corresponding protected or immobilised polypeptide is subjected to deprotection or removal from a solid support.

The nucleic acid sequences and polypeptides according to the invention may be used to prepare vaccine compositions using methods well known in the art of vaccine manufacture.

Traditional vaccine formulations may comprise one or more polypeptides according to the invention together, where appropriate, with one or more suitable adjuvants eg. aluminium hydroxide, muramyl dipeptide, mineral or vegetable oils, novosomes, liposomes, saponins, Quil-A, Q5-21, Matrix or pluronics, in the presence of one or more pharmaceutically acceptable carriers or diluents. Suitable carriers include liquid media such as saline solution appropriate for use as vehicles to introduce the polypeptides into a subject. Additional components such as preservatives may be included and the vaccines may also comprise other antigenic components such as other FIV antigens eg. other FIV proteins and/or polypeptides or other feline virus, eg. feline leukaemia virus, antigens, to forms a multivalent-vaccine. Immune stimulating complexes (ISCOMS Morein et al., Nature, 308: 457-460, 1984) have recently been found to be particularly effective as adjuvants in vaccine preparations and are particularly attractive in presenting antigens which comprise "transmembrane" portions.

Such vaccine compositions form a further aspect of the present invention, and the invention can thus be seen also to provide a composition for combatting FIV, preferably a vaccine composition for stimulating an immune response in an animal against FIV, said composition comprising one or more synthetic polypeptides comprising an amino acid sequence selected from

| FIV envelope protein | | |
|---|---|---|
| | Residue Numbers | Sequence |
| SEQ ID. NO. | | |
| 1 | 377-417 | CQRTQSQPGLWLRAISSWKQRNRWEWRPDFKSKKMKISLQC; and/or |
| 2 | 462-490 | CRWNVGDNTSLIDTCGETQNVSGANPVDC; and/or |
| 3 | 354-377 | CGWWNQIAYYNSCRWESTDVKFHC; and/or |
| 5 | 417-445 | CNSTQNLTFAMRSSGDYGEVTGAWIEFGC; and/or |
| 6 | 320-339 | CTDPLQIPLINYTFGPNQTC; and/or |
| 7 | 161-190 | LFIGVGIYLGTAKAQVVWRLPPLVVPVEES; and/or |
| 8 | 248-267 | CRRGRIWKRWNETITGPSGC; and/or |
| 10 | 716-736 | NMTINQTIWNHGNITLGEWYN, |

together with, optionally, one or more adjuvants, and a pharmaceutically acceptable carrier or diluent.

An alternative favourable way of presenting the vaccine according to the invention, is to administer to the animal an expression vector, capable of expressing the polypeptides in question such that the immunogenic polypeptides are expressed in situ in the animal. Such "in situ" expression of the antigenic polypeptides has been found to present the immunogen to the immune system of the animal in a particularly favourable manner.

A further preferred aspect of the invention thus includes a composition for combatting FIV, preferably a vaccine composition for stimulating an immune response in a mammal against FIV, said composition comprising an expression vector, or host cell having inserted therein a nucleic acid molecule according to this invention as defined above for stimulation of an immune response directed against polypeptides encoded by the inserted nucleotide sequence, together with one or more pharmaceutically acceptable carriers or diluents.

Expression vectors suitable for such use are known in the art and include in particular viral vectors, notably pox virus vectors. Vectors worthy of particular mention include the vaccinia virus, fowl pox virus, canary pox virus, feline rhinotracheitis, feline calicivirus, feline panleukopenia, adenovirus and bacteria (eg. Salmonella) as described for example by Ada et al., Vaccine 8: 425-437, 1990. Advantageously a fusion protein may be expressed in such a system, where the heterologous protein part (the "carrier") comprises all or a portion of a transmembrane protein (eg. influenza virus HA). When expressed in eukaryotic cells infected with such a recombinant virus, the fusion protein is generally glycosylated and transported to the cell surface through which it protrudes thereby presenting the synthetic polypeptides (or their epitopes) on the outside of the cell surface.

Administration of a composition eg. a vaccine composition according to the invention may take place by any of the conventional routes, eg. orally or parenterally such as by subcutaneous, intravenous, intramuscular or intradermal injection, optionally at intervals eg. two injections at a 7-42 day interval. Typically a peptide is administered in an amount of 1 to 1000 µg per dose, more preferably 2 to 100 µg per dose, by either the oral or the parenteral route. Where a recombinant viral vector is administered as a vaccine, the routes of administration and dose may be the same.

In the various aspects of this invention discussed above, the animal is preferably a mammal, and most preferably a cat or a kitten.

We have shown that synthetic polypeptides according to the invention may be recognised by antisera from cats positive for FIV. Accordingly, such polypeptides may be used in the detection of antibodies against FIV and may thus form the basis of a diagnostic test for FIV, in particular to identify FIV infected cats which have seroconverted.

A further aspect of the invention thus provides a method for detecting antibody to FIV, said method comprising contacting one or more synthetic polypeptides according to the present invention with a sample comprising said antibody, and detecting the presence of a complex formed between said polypeptide and said antibody.

Detection of such complex formation may be accomplished using standard immunoassay techniques such as ELISA for example.

The sample may be any clinical or biological sample and may comprise for example a body fluid or solid (eg. a blood, plasma, serum, urine or tissue sample). Generally however the sample will be a blood-derived sample.

Conveniently the reagents required to perform the diagnostic test will be supplied in kit form and this represents a further aspect of the invention.

Accordingly, the invention can also be seen to provide a test kit for use in detecting antibodies to FIV in a sample, comprising one or more synthetic polypeptides according to the invention and means for detecting complex formation between said polypeptide and said antibody.

Such detecting means may conveniently comprise one or more labelled eg. colorimetrically labelled secondary antibodies or colormetrically labelled forms of said synthetic polypeptides.

Monoclonal antibodies against the above FIV polypeptides may also be of use in diagnostic systems, for example in labelled forms, and constitute a further feature of this invention.

The invention will now be discussed in more detail in the following non-limiting Examples with reference to the accompanying drawings in which:

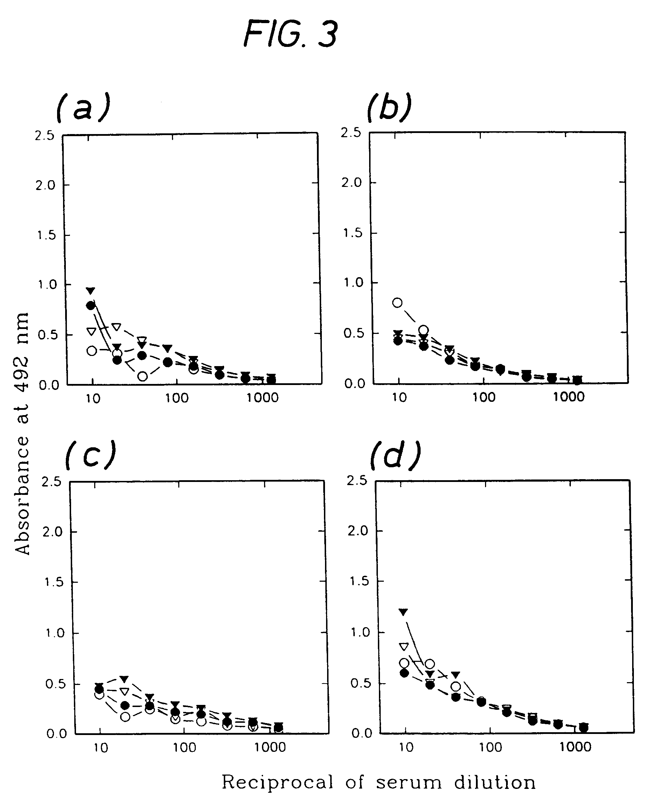

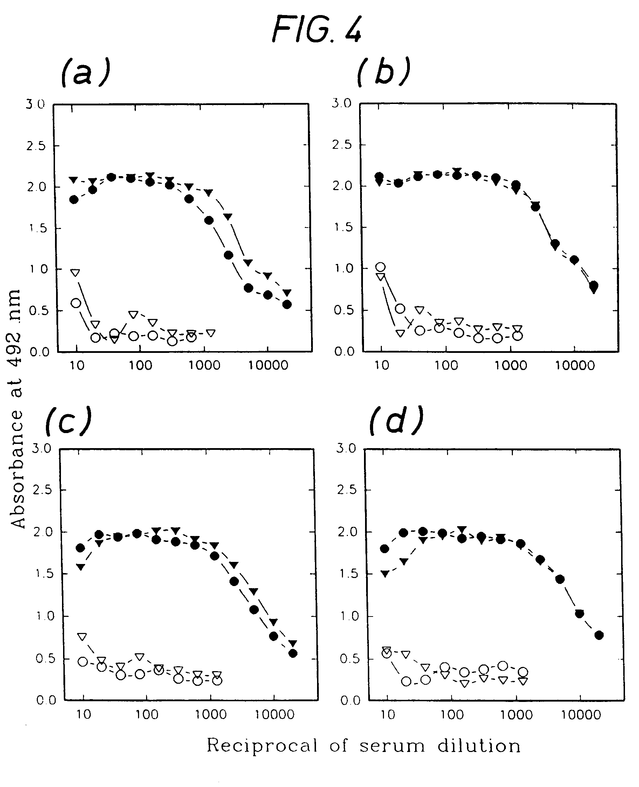

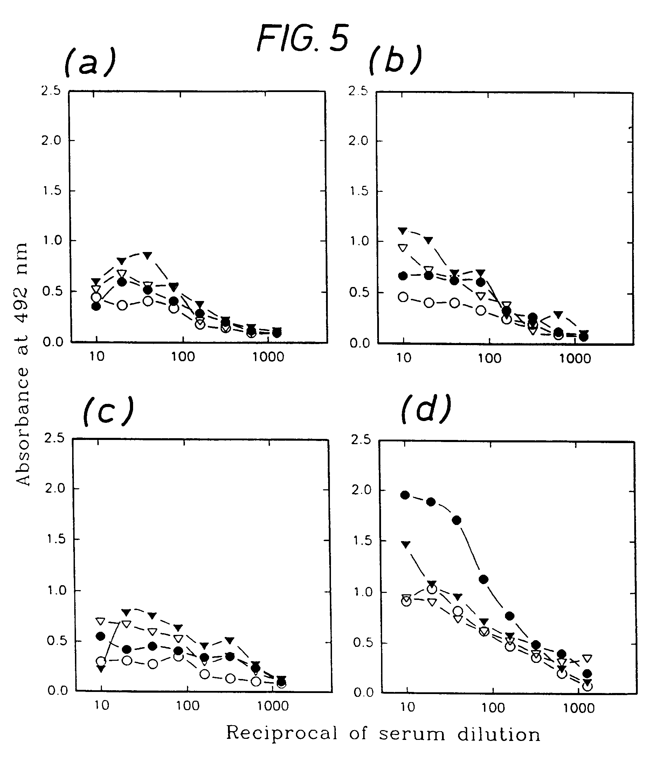

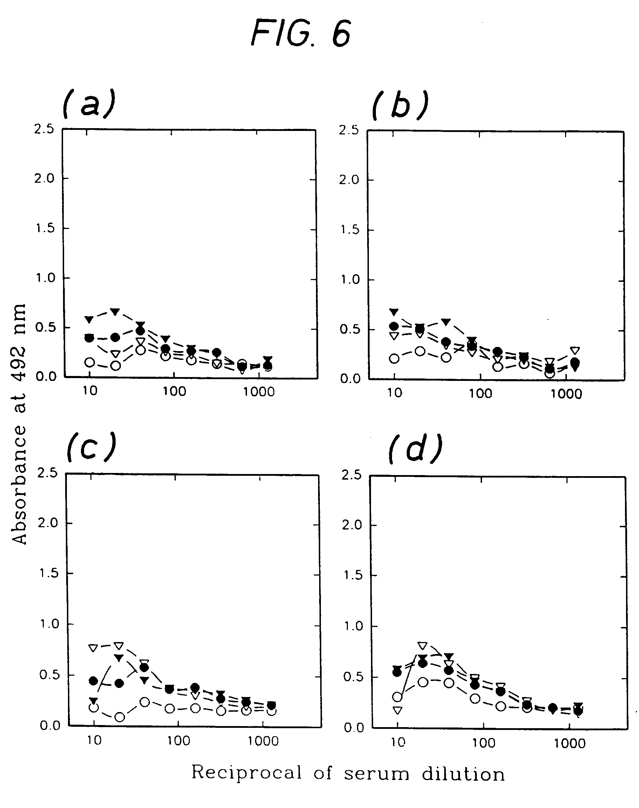

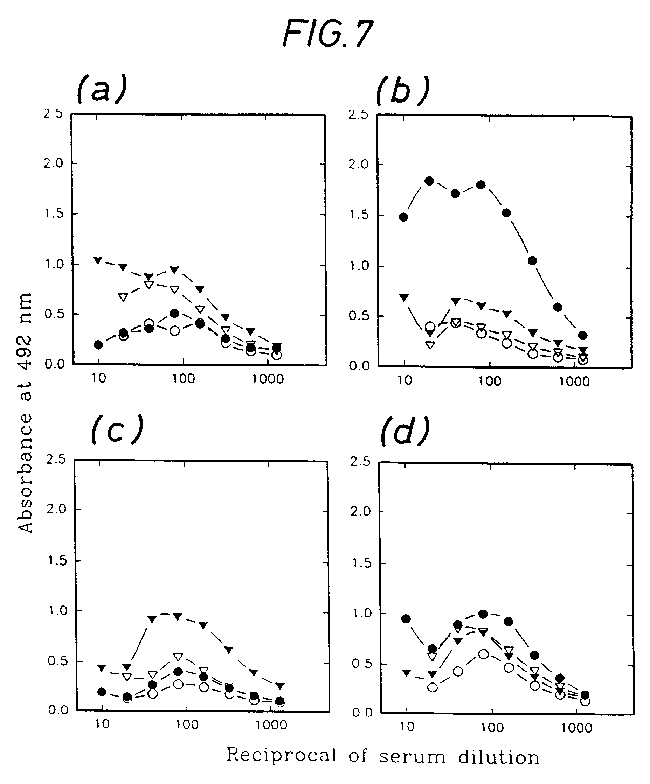

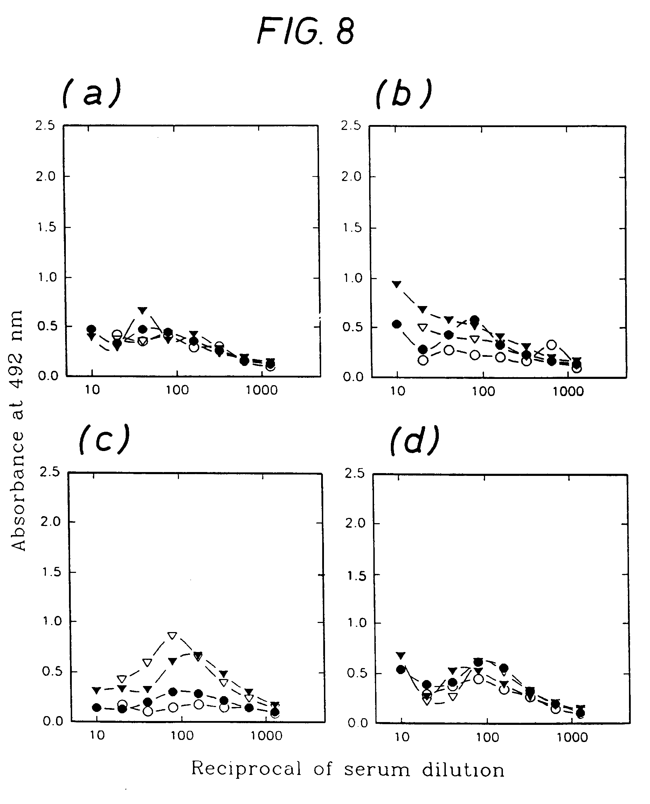

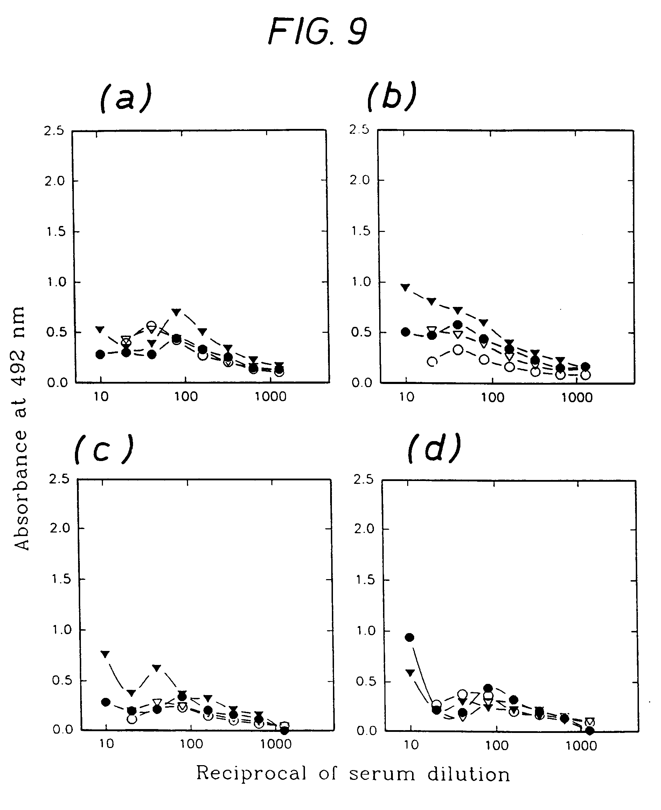

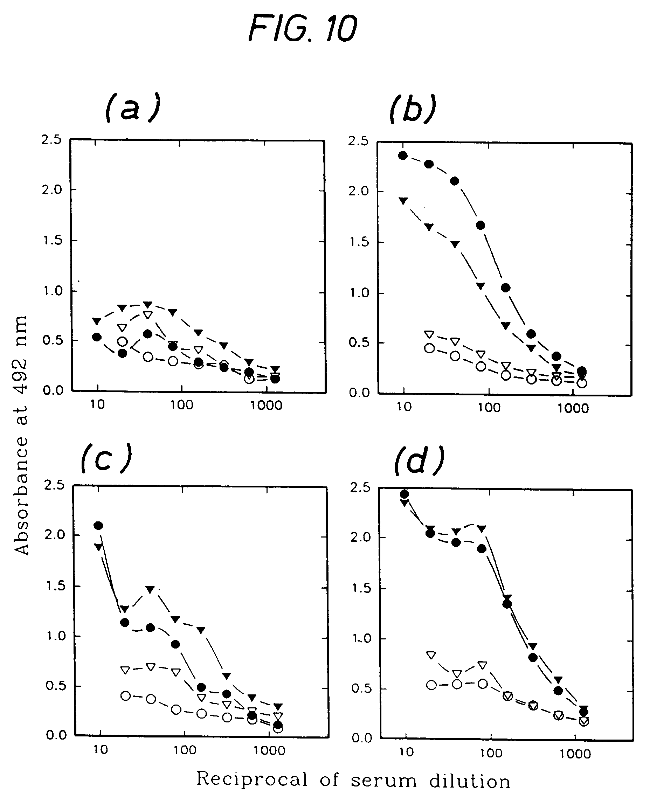

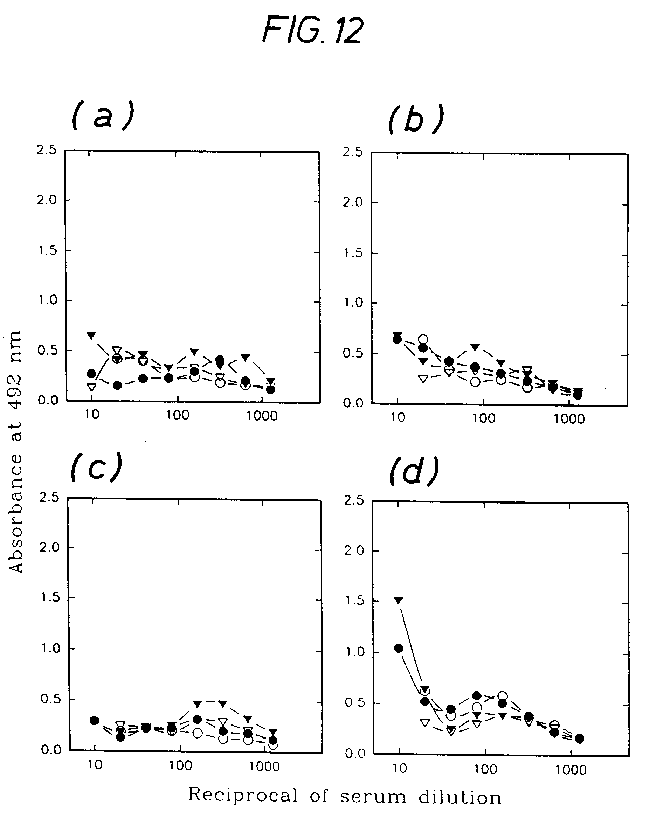

Figures 3 to 12 show the antibody response to FIV polypeptides 1 to 10, respectively, in antisera from FIV-infected cats as measured by indirect ELISA.

In these figures the abscissa shows reciprocal of serum dilution, the ordinate Absorbance at 492 nm;

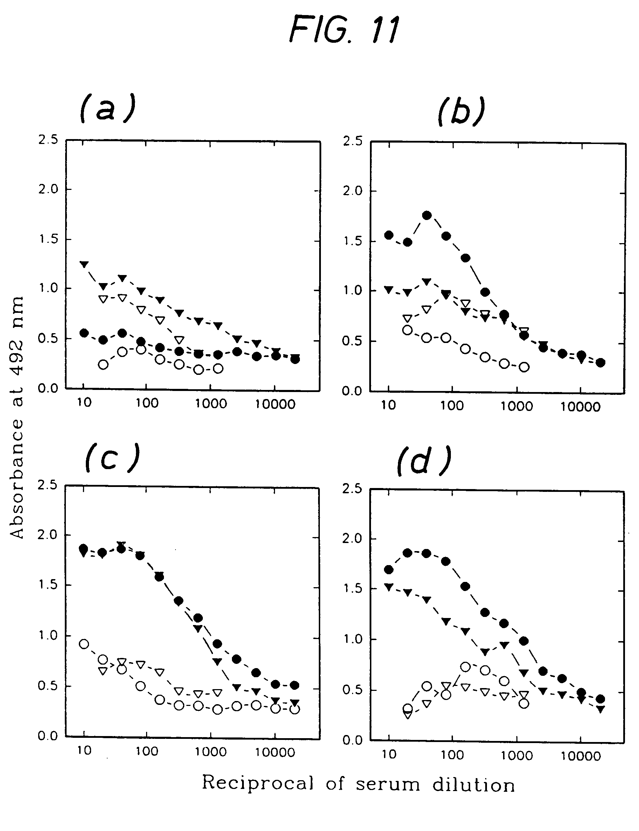

the symbols represent, in sections (a) to (d) of the Figures respectively (other than in section (c) of Figure 11):
(a)
   ○ pre-bleed cat A5
   ∇ pre-bleed cat A6
   ● immune bleed cat A5
   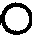 immune bleed cat A6
(b)
   ○ pre-bleed cat A7
   ∇ pre-bleed cat A9
   ● immune bleed cat A7
   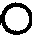 immune bleed cat A9
(c)
   ○ pre-bleed cat A10
   ∇ pre-bleed cat A11
   ● immune bleed cat A10
   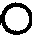 immune bleed cat A11
(d)
   ○ pre-bleed cat A12
   ∇ pre-bleed cat A34
   ● immune bleed cat A12
   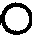 immune bleed cat A34

In section (c) of Figure 11 the symbols represent:
○ normal cat serum
∇ pre-bleed cat A11
● immune bleed cat A10
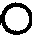 immune bleed cat A11

### EXAMPLE 1

### Materials and Methods

### Peptides

Peptides were synthesized by Dr. James Tam of the Rockerfeller University, USA using solid phase chemistry method developed by Merrifield (1963) as follows:

| | Residue Numbers | Sequence |
|---|---|---|
| SEQ ID. NO. | | |
| 1 | 377-417 | CQRTQSQPGLWLRAISSWKQRNRWEWRPDFKSKKMKISLQC; and/or |
| 2 | 462-490 | CRWNVGDNTSLIDTCGETQNVSGANPVDC; and/or |
| 3 | 354-377 | CGWWNQIAYYNSCRWESTDVKFHC; and/or |
| 4 | 302-320 | CLTGGKMLYNKVTKQLSYC; and/or |
| 5 | 417-445 | CNSTQNLTFAMRSSGDYGEVTGAWIEFGC; and/or |
| 6 | 320-339 | CTDPLQIPLINYTFGPNQTC; and/or |
| 7 | 161-190 | LFIGVGIYLGTAKAQVVWRLPPLVVPVEES; and/or |
| 8 | 248-267 | CRRGRIWKRWNETITGPSGC; and/or |
| 9 | 601-620 | VMEYKARRKRAAIHVMLALA; and/or |
| 10 | 716-736 | NMTINQTIWNHGNITLGEWYN. |

Each peptide was vigorously purified by reverse phase HPLC and characterised by amino acid analysis and mass spectrometric analysis. Additionally non-natural cysteine residues were added to the carboxyl-terminus of peptides 7, 9 and 10 (SEQ ID NOS: 7, 9 and 10) to facilitate coupling to a protein carrier. Furthermore, amino-terminal cysteines in each peptide were left protected with an acetamide group as Cys (Acm) to enable specific carrier linkage through the carboxyl-terminal cysteine. It also provided the opportunity for producing the polymers or cyclic peptides by activating the Cys (Acm) and thus removing the Acm group. Sequences are based on a combination of the UK8 and Petaluma sequences. Peptide 1 (SEQ ID NO: 1) contains a non-natural Met at position 411 and peptide 9 (SEQ ID NO: 9) contains a non-natural Ala at position 607. Position 421 in peptide 5 (SEQ ID NO: 5) contains a residue from FIV Dutch 19k1 and not UK8 or Petaluma.

### Antisera

Antisera collected from 8 cats before and after exposure to FIV infection were provided by Prof O. Jarrett of the Feline virus unit, Glasgow University. The code numbers of the cats were A5, A6, A7, A9, A10, A11, A12 and A34.

### Anti peptide Assay

A modification of the indirect ELISA technique described by Voller & Bidwell Br.J. Exp. Path., 57: 243-247, 1976, was used to assay anti-peptide antibody responses. Briefly, microplates were coated overnight at room temperature with uncoupled synthetic peptide at a concentration of 10 µg/ml. The plates were washed and test serum samples at a range of doubling dilutions from 1:10 were added. After incubation for 1 hour at 37°, plates were washed and anti-cat IgG-peroxidase conjugate was added. After a further hour at 37°C, the plates were washed and an enzyme substrate (0.04% o-phenylenediamine +0.004% hydrogen peroxide in phosphate/citrate buffer) added. The resulting colour development was stopped with 12.5% sulphuric acid after 3-5 minutes and the absorbance at 492 nm measured in a Titertek Multiskan plus (Flow Laboratories, Irvine, Ayrshire, U.K.).

The A₄₉₂ values obtained from doubling dilutions of post-inoculation samples were plotted against the log₁₀ reciprocal antiserum dilution. The results reported are the means of two tests.

### Results

Graphs showing the reactivity of the cat antisera against each of the 10 peptides are presented in Figures 3 to 12. The results of these graphs are summarised in Table 1. This table shows that all peptides had a low level of serological reactivity against at least one, or more, of the FIV positive antisera. Furthermore, 5 of these 10 peptides (FIV 2, 3, 5, 8 and 9 (SEQ ID NOS: 2, 3, 5, 8 and 9)) had significant reactivity against one, or more of the antisera. Particularly notable in this respect was peptide 2 (SEQ ID NO: 2) which reacted well with all of the 8 cat antisera, and peptides 8 and 9 (SEQ ID NOS: 8 and 9) which reacted strongly with 6 of the 8 and 5 of the 8 antisera respectively. The overall anti-peptide titres detected in many of the antisera were also very high e.g. >1:10,000 against peptide 2 and >1:1000 against peptides 8 and 9 (SEQ ID NOS. 8 and 9). No single cat antiserum reacted with all of the peptides although A7, A10, A11, A12 and A34 reacted strongly with 3 or more of the peptides.

These results demonstrate that each of the above 10 peptides selected from the envelope protein of FIV are recognised to some extent by at least one or more of the FIV positive antisera. This recognition is highly significant for 5 of the peptides and in the case of three of these the majority of the cat sera tested are positive. These results indicate therefore that these peptides or combinations of the peptides would be useful in the detection of sero positive cats exposed to FIV infection. Furthermore, the use of such peptides as the basis of a vaccine against FIV is also indicated.

**Table 1**

| Reactivity of FIV Peptides against a range of FIV positive cat antisera as measured by indirect ELISA | | | | | | | | |
|---|---|---|---|---|---|---|---|---|
| Peptide Number | Cat Antiserum Number | | | | | | | |
| | A5 | A6 | A7 | A9 | A10 | A11 | A12 | A34 |
| FIV1 | +/- | +/- | - | - | - | - | - | +/- |
| FIV2 | ++++ | ++++ | ++++ | ++++ | ++++ | ++++ | ++++ | ++++ |
| FIV3 | - | +/- | +/- | +/- | - | - | +++ | +/- |
| FIV4 | - | +/- | - | - | - | - | - | - |
| FIV5 | - | +/- | ++++ | +/- | - | ++ | + | - |
| FIV6 | - | - | - | +/- | - | - | - | - |
| FIV7 | - | - | + | +/- | + | - | - | - |
| FIV8 | - | +/- | ++++ | ++++ | +++ | +++ | ++++ | ++++ |
| FIV9 | - | +/- | +++ | +/- | +++ | +++ | ++++ | +++ |
| FIV10 | - | - | - | - | - | - | - | +/- |
| Key +/- Indication of activity above pre-bleed values only at 1/10 dilution + OD 0.1-0.3 above pre-bleed ++ OD 0.4-0.6 above pre-bleed +++ OD 0.7-1.0 above pre-bleed ++++ OD >1.1 above pre-bleed | | | | | | | | |

### EXAMPLE 2

### Further serological screening assays on cat antisera

### Introduction

Following the initial encouraging results obtained by screening 8 antisera from cats infected with the UK (Glasgow) 8 virus against the 10 FIV peptides as described in Example 1, 5 of the most reactive peptides were selected for more extensive screening. The five peptides selected were peptides 2, 3, 5, 8 and 9 (SEQ ID NOS. 2, 3, 5, 8 and 9).

The cat antisera used were a panel of 59 antisera from cats infected with UK isolates (made up of 6 antisera from experimentally infected cats held at Bristol University, and 53 antisera consisting of field isolates and experimentally infected cats from Glasgow University), 36 antisera from cats infected with Dutch isolates (serial bleeds from 4 experimentally infected cats Nos. 11 to 14) and 3 antisera from cats infected with USA isolates of FIV (5040, 5039 and 8917-6).

### Results

The overall results obtained from screening the 59 antisera from cats exposed to UK isolates of FIV were that 94% showed a positive reaction against peptide 2 (SEQ ID NO: 2); 69.6% against peptide 3 (SEQ ID NO: 3); 76.8% against peptide 5 (SEQ ID NO: 5); 88.4% against peptide 8 (SEQ ID NO: 8); and 70% against peptide 9 (SEQ ID NO: 9).

Sera collected from cats infected with Dutch isolates showed a strong response to peptide 2 (SEQ ID NO: 2) post-infection (Table 2). Lower reactivity was also seen to the other 4 peptides in antisera from two of the cats (12 and 14). Of the three antisera from cats infected with US isolates, two (5039 and 5040) showed marked responses to the majority of the 5 peptides, while one was only weakly reactive with peptides 2 and 9 (SEQ ID NOS 2 and 9) (Table 2).

### Conclusions

These results demonstrate that antisera collected from cats infected with a broad range of FIV isolates of both European and USA origin show significant responses against peptides FIV 2, 3, 5, 8 and 9 (SEQ ID NOS: 2, 3, 5, 8 and 9). This confirms and extends the results presented in Example 1 and supports utility of the peptides for diagnostic as well as vaccine purposes.

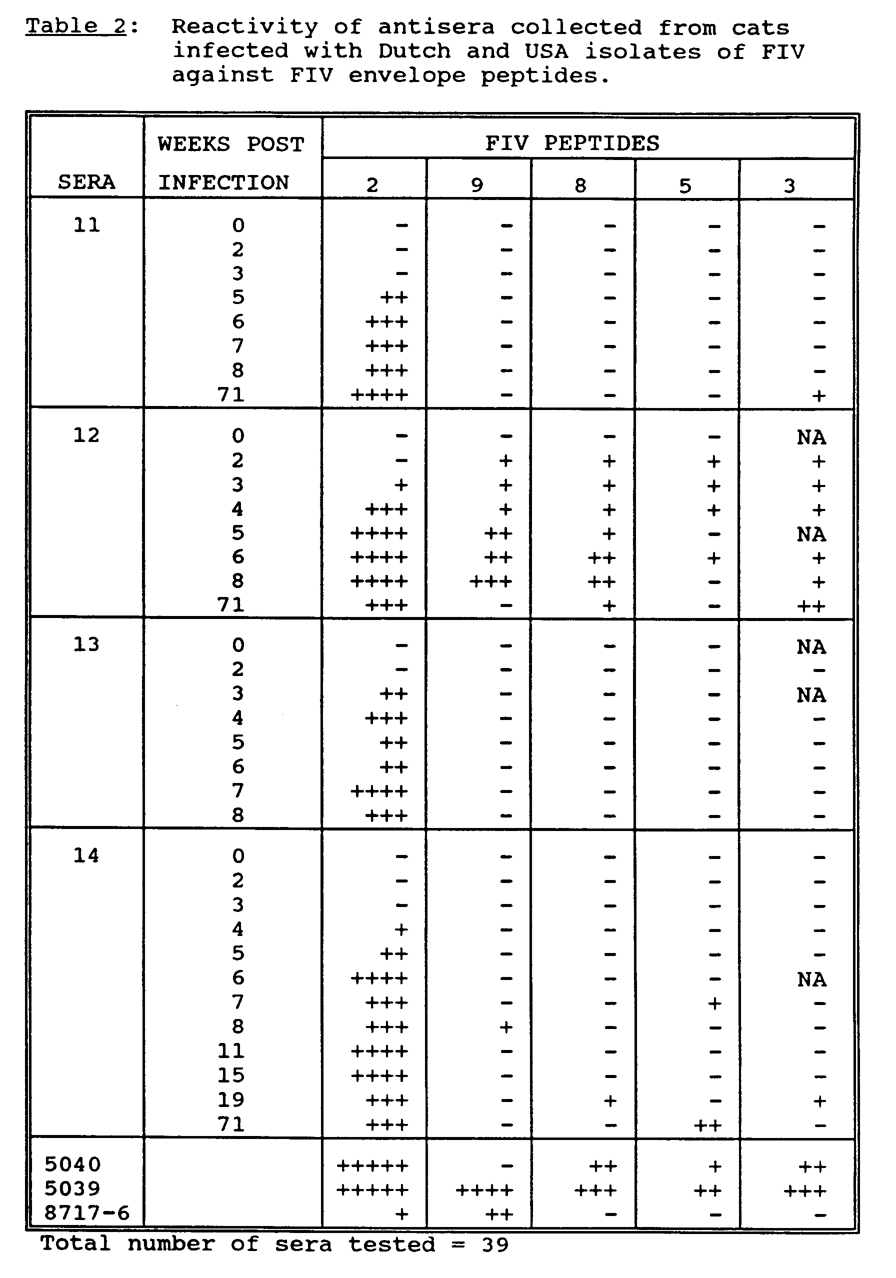

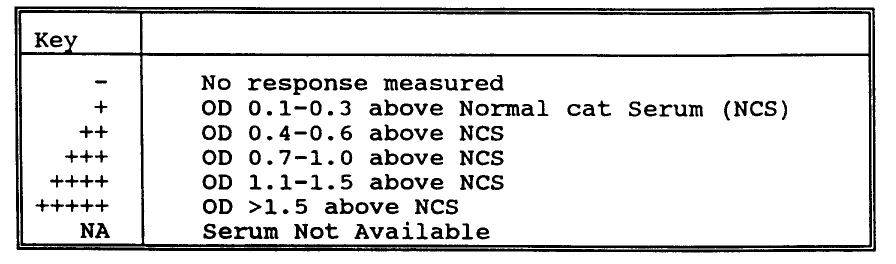

### EXAMPLE 3

### Production and characterisation of antibodies to FIV peptides

### Introduction

In order to assess the immunogenic potential of the 10 FIV peptides, each peptide was coupled to a protein carrier (Keyhole Limpet Haemocyanin - KLH) and 50 µg was inoculated subcutanteously into 10 groups of 2 guinea pigs and 2 rabbits using a 23g needle with a 0.5ml dose. All animals were boosted as above at days 42 and 48, and antisera collected at day 108 were analysed for anti-homologous peptide, anti-KLH and anti-recombinant FIV gp130 by ELISA, and anti-FIV gp130 in a Western blot.

### Results

Results obtained for guinea pigs and rabbits are presented in Tables 3 and 4 respectively.

It can be seen from Table 3 that high titre (≥1:10240) anti-peptide antisera were produced in guinea pigs to 8 of the 10 peptides. Peptides 7 and 9 (SEQ ID NOS: 7 and 9) failed to elicit detectable peptide responses although high titre responses against KLH were produced. The reason for this is unclear. Furthermore, antisera to peptides 2, 5 and 6 (SEQ ID NOS: 2, 5 and 6) also showed reactivity with recombinant FIV gp130 in ELISAs and Western blots. A low activity against gp130 in ELISA was also seen with peptide 9 (SEQ ID NO: 9) antisera in spite of the fact that no anti-peptide antibodies were detectable.

Table 3 shows that high titre (≥ 1:2560) rabbit anti-peptide antisera were produced to 6 of the 10 peptides (SEQ ID NOS: 1, 2, 4, 5, 6 and 9) while lower titre antisera (1:40 to 1:320) were produced to the remaining 4 peptides (SEQ ID NOS: 3, 7, 8 and 10). With only one exception (rabbit 11, titre 1:640) responses to KLH were universally high (≥1:10240). ELISA reactivity against FIV gp130 was detected in 12 of the antisera which covered 8 of the 10 peptides tested. Only peptides 1 and 7 (SEQ ID NOS: 1 and 7) failed to produce any anti-FIV gp130 activity, as detected by ELISA, although peptide 7 (SEQ ID NO: 7) antisera did show some activity against gp130 in a Western blot. Interestingly, some anti-peptide antisera with activity detectable against FIV gp130 in an ELISA (3, 8, 9, and 10) did not react with denatured gp130 in a Western blot.

### Conclusions

These results demonstrate that following chemical conjugation of the peptides to a protein carrier it is possible to produce anti-peptide antibodies to all 10 of the FIV peptides. Furthermore, selected sera against 9 of the 10 peptides demonstrate some anti-FIV envelope protein activity. Although the functional significance of these results is not known they do indicate utility of the peptides for FIV vaccine development.

**Table 3**

| Antibody responses at day 108 in guinea-pigs immunised with KLH coupled FIV peptides 1-10 | | | | | |
|---|---|---|---|---|---|
| | | | | | WESTERN BLOT |
| GROUP/PEPTIDE | ANIMAL NUMBER | Anti-homologous peptide | Anti-KLH | Anti-FIV gp130 | Anti-FIV gp130 |
| 1 | 1 | >1:10240 | >1:10240 | - | - |
| | 2 | >1:10240 | >1:10240 | - | - |
| 2 | 1 | >1:10240 | >1:10240 | >1:1280 | ++ |
| | 2 | NA | NA | NA | NA |
| 3 | 1 | 1:10240 | >1:10240 | - | - |
| | 2 | NA | NA | NA | NA |
| 4 | 1 | >1:10240 | >1:10240 | - | - |
| | 2 | 1:1280 | >1:10240 | - | - |
| 5 | 1 | >1:10240 | >1:10240 | 1:428 | ++ |
| | 2 | >1:10240 | >1:10240 | 1:169 | ++ |
| 6 | 1 | >1:10240 | >1:10240 | - | + |
| | 2 | >1:10240 | >1:10240 | 1:78 | + |
| 7 | 1 | - | >1:10240 | - | - |
| | 2 | - | >1:10240 | - | - |
| 8 | 1 | >1:10240 | >1:10240 | - | - |
| | 2 | >1:10240 | >1:10240 | - | - |
| 9 | 1 | - | 1:10240 | 1:16 | - |
| | 2 | NA | NA | NA | NA |
| 10 | 1 | 1:10240 | >1:10240 | - | - |
| | 2 | 1:10240 | >1:10240 | - | - |
| NA Not available (animal died) - No response above Day 0 ++ Strong 130kd band + Light 130kd band | | | | | |

**Table 4**

| Antibody responses at day 108 in rabbits immunised with KLH coupled FIV peptides 1-10 | | | | | |
|---|---|---|---|---|---|
| | | | | | WESTERN BLOT |
| GROUP/PEPTIDE | ANIMAL NUMBER | Anti-homologous peptide | Anti-KLH | Anti-FIV gp130 | Anti-FIV gp130 |
| 1 | 5 | NA | NA | NA | NA |
| | 3 | >1:10240 | >1:10240 | - | - |
| 2 | 6 | NA | NA | NA | NA |
| | 12 | >1:10240 | 1:10240 | 1:768 | +++ |
| 3 | 13 | - | >1:10240 | 1:17 | - |
| | 8 | 1:40 | 1:10240 | 1:36 | - |
| 4 | 17 | 1:5120 | >1:10240 | - | + |
| | 18 | - | 1:10240 | 1:59 | + |
| 5 | 19 | >1:10240 | >1:10240 | 1:262 | +++ |
| | 20 | >1:10240 | >1:10240 | 1:46 | ++ |
| 6 | 4 | >1:10240 | >1:10240 | 1:74 | + |
| | 11 | 1:320 | 1:640 | 1:39 | + |
| 7 | 14 | 1:40 | >1:10240 | - | + |
| | 16 | - | >1:10240 | - | + |
| 8 | 7 | NA | NA | NA | NA |
| | 9 | 1:320 | >1:10240 | 1:17 | - |
| 9 | 1 | >1:10240 | >1:10240 | 1:10 | - |
| | 15 | 1:2560 | 1:10240 | - | - |
| 10 | 10 | 1:320 | >1:10240 | 1:12 | - |
| | 2 | - | >1:10240 | 1:67 | - |
| NA Not available (animal died) - No response above Day 0 +++ Strong 130kd band ++ Medium 130kd band + Light 130kd band | | | | | |

## Claims

1. A synthetic polypeptide comprising an amino acid sequence selected from:
| FIV envelope protein | | |
|---|---|---|
| | Residue Numbers | Sequence |
| SEQ ID. NO. | | |
| 1 | 377-417 | CQRTQSQPGLWLRAISSWKQRNRWEWRPDFKSKKMKISLQC; and/or |
| 2 | 462-490 | CRWNVGDNTSLIDTCGETQNVSGANPVDC; and/or |
| 3 | 354-377 | CGWWNQIAYYNSCRWESTDVKFHC; and/or |
| 5 | 417-445 | CNSTQNLTFAMRSSGDYGEVTGAWIEFGC; and/or |
| 6 | 320-339 | CTDPLQIPLINYTFGPNQTC; and/or |
| 7 | 161-190 | LFIGVGIYLGTAKAQVVWRLPPLVVPVEES; and/or |
| 8 | 248-267 | CRRGRIWKRWNETITGPSGC; and/or |
| 10 | 716-736 | NMTINQTIWNHGNITLGEWYN; |
or an antigenic fragment, functionally-equivalent variant retaining immunogenic activity or pharmaceutically acceptable salt thereof.

2. A synthetic polypeptide as claimed in claim 1 for use in combatting FIV.

3. A synthetic polypeptide as claimed in claim 1 or claim 2, in the form of a fusion protein comprising an additional polypeptide fused to said amino acid sequence, or which is coupled to a carrier protein or polypeptide.

4. Use of a synthetic polypeptide as defined in any one of claims 1 to 3, in the preparation of a composition for combatting FIV.

5. A nucleic acid molecule comprising a nucleotide sequence comprising a region of the FIV env gene encoding residues of the FIV envelope protein selected from:
Residues
(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a portion thereof encoding an antigenic fragment of FIV, or a sequence which is degenerate or has greater than 60% sequence homology therewith or which hybridises under conditions of high stringency with any such aforesaid sequence, for use in combatting FIV.

6. A nucleic acid molecule comprising a first nucleotide sequence corresponding to a region of the FIV env gene encoding residues of the FIV envelope protein selected from:
Residues
(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a portion thereof encoding an antigenic fragment of FIV, or a sequence which is degenerate or has greater than 60% sequence homology therewith or which hybridises under conditions of high stringency with any such aforesaid sequence, together with at least one additional nucleotide sequence flanking said first nucleotide sequence, the additional nucleotide sequence comprising no more than 900 bases.

7. A nucleic acid molecule as defined in claim 6 for use in combatting FIV.

8. Use of a nucleic acid molecule as defined in claim 5 or claim 6 in the preparation of a composition for combatting FIV.

9. The use of a nucleic acid molecule comprising a nucleotide sequence encoding a polypeptide capable of raising neutralising antibodies against FIV which sequence incorporates one or more antigenic determinant-encoding regions from the regions of the FIV env gene encoding residues of the FIV envelope protein selected from:
(1) 377-417; and/or
(2) 462-490; and/or
(3) 354-377; and/or
(5) 417-445; and/or
(6) 320-339; and/or
(7) 161-190; and/or
(8) 248-267; and/or
(10) 716-736;
or a sequence which is degenerate, has greater than 60% sequence homology therewith or which hybridises under conditions of high stringency with any such aforesaid sequence, for the preparation of a composition for combatting FIV.

10. An expression or cloning vector comprising a nucleic acid molecule comprising a nucleotide sequence as defined in claim 5.

11. A host cell or transgenic organism containing a nucleic acid molecule comprising a nucleotide sequence as defined in claim 5 or an expression vector as claimed in claim 10.

12. A method for preparing a synthetic polypeptide as defined in any one of claims 1 to 3, which comprises culturing host cells as defined in claim 11, under conditions whereby said polypeptide is expressed, and recovering said polypeptide thus produced.

13. A process for the preparation of synthetic polypeptides as defined in any one of claims 1 to 3 in which a corresponding protected or immobilised polypeptide is subjected to deprotection or removal from a solid support.

14. A composition for combatting FIV, comprising one or more synthetic polypeptides as defined in any one of claims 1 to 3, or an expression vector or host cell having inserted therein a nucleic acid molecule comprising a nucleotide sequence as defined in claim 5, for stimulation of an immune response directed against polypeptides encoded by the inserted nucleic acid molecule, together with one or more pharmaceutically carriers or diluents.

15. A composition as claimed in claim 14, additionally comprising one or more other antigenic FIV proteins and/or polypeptides.

16. Antibodies binding specifically to a synthetic polypeptide as defined in any one of claims 1 to 3.

17. A method for detecting antibody to FIV, said method comprising contacting one or more synthetic polypeptides as defined in any one of claims 1 to 3 with a sample comprising said antibody, and detecting the presence of a complex formed between said polypeptide and said antibody.

18. A test kit for use in detecting antibodies to FIV in a sample, comprising one or more synthetic polypeptides as defined in any one of claims 1 to 3 and means for detecting complex formation between said polypeptide and said antibody.

## Patentansprüche

1. Synthetisches Polypeptid, umfassend eine Aminosäuresequenz, ausgewählt aus:
| **KIV-Hüllprotein** | | |
|---|---|---|
| | **Zahl der Reste** | **Sequenz** |
| **SEQ ID Nr.** | | |
| 1 | 377-417 | CQRTQSQPGLWLRAISSWKQRNRWEWRPDFKSKKMKISLQC |
| | | und/oder |
| 2 | 462-490 | CRWNVGDNTSLIDTCGETQNVSGANPVDC |
| | | und/oder |
| 3 | 354-377 | CGWWNQIAYYNSCRWESTDVKFHC |
| | | und/oder |
| 5 | 417-445 | CNSTQNLTFAMRSSGDYGEVTGAWIEFGC |
| | | und/oder |
| 6 | 320-339 | CTDPLQIPLINYTFGPNQTC |
| | | und/oder |
| 7 | 161-190 | LFIGVGIYLGTAKAQVVWRLPPLVVPVEES |
| | | und/oder |
| 8 | 248-267 | CRRGRIWKRWNETITGPSGC |
| | | und/oder |
| 10 | 716-736 | NMTINQTIWNHGNITLGEWYN |
oder ein antigenes Fragment, eine funktionell-äquivalente Variante, die die immunogene Aktivität beibehält, oder ein pharmazeutisch annehmbares Salz davon.

2. Synthetisches Polypeptid nach Anspruch 1 zur Verwendung bei der KIV-Bekämpfung.

3. Synthetisches Polypeptid nach Ansprüchen 1 oder 2 in Form eines Fusionsproteins, umfassend ein zusätzliches Polypeptid, das an diese Aminosäuresequenz gebunden, oder das an ein Trägerprotein oder ein Polypeptid gekoppelt ist.

4. Verwendung eines synthetischen Polypeptids nach einem der Ansprüche 1 bis 3 bei der Herstellung einer Zusammensetzung zur KIV-Bekämpfung.

5. Nucleinsäuremolekül, umfassend eine Nucleotidsequenz, die einen Bereich der KIV env Gen codierenden Reste des KIV-Hüllproteins umfaßt, ausgewählt aus:
**Reste**
(1) 377-417 und/oder
(2) 462-490 und/oder
(3) 354-377 und/oder
(5) 417-445 und/oder
(6) 320-339 und/oder
(7) 161-190 und/oder
(8) 248-267 und/oder
(10) 716-736,
oder einen Teil davon, der ein antigenes KIV-Fragment codiert oder eine Sequenz, die degeneriert ist oder eine Sequenzhomologie mit dieser von mehr als 60 % hat oder die unter stark stringenten Bedingungen mit einer der vorgenannten Sequenz hybridisiert, zur Verwendung bei der KIV-Bekämpfung.

6. Nucleinsäuremolekül, umfassend eine erste Nucleotidsequenz, die einem Bereich der KIV env Gen codierenden Reste des KIV-Hüllproteins entsprechen, ausgewählt aus:
**Reste**
(1) 377-417 und/oder
(2) 462-490 und/oder
(3) 354-377 und/oder
(5) 417-445 und/oder
(6) 320-339 und/oder
(7) 161-190 und/oder
(8) 248-267 und/oder
(10) 716-736,
oder einen Teil davon, der ein antigenes KIV-Fragment codiert oder eine Sequenz, die degeneriert ist oder eine Sequenzhomologie mit dieser von mehr als 60 % hat oder die unter stark stringenten Bedingungen mit jeder der vorgenannten Sequenz hybridisiert, zusammen mit mindestens einer zusätzlichen Nucleotidsequenz, die von dieser ersten Nucleotidsequenz flankiert ist, wobei die zusätzliche Nucleotidsequenz nicht mehr als 900 Basen umfaßt.

7. Nucleinsäuremolekül nach Anspruch 6 zur Verwendung bei der KIV-Bekämpfung.

8. Verwendung eines Nucleinsäuremoleküls nach Anspruch 5 oder 6 bei der Herstellung einer Zusammensetzung zur KIV-Bekämpfung.

9. Verwendung eines Nucleinsäuremoleküls, umfassend eine Nucleotidsequenz, die ein Polypeptid codiert, das neutralisierende Antikörper gegen KIV entwickeln kann, wobei die Sequenz ein oder mehrere antigene Determinant-codierende Bereiche von den Bereichen der KIV env-Gen codierenden Reste des KIV-Hüllproteins, ausgewählt aus
**Reste**
(1) 377-417 und/oder
(2) 462-490 und/oder
(3) 354-377 und/oder
(5) 417-445 und/oder
(6) 320-339 und/oder
(7) 161-190 und/oder
(8) 248-267 und/oder
(10) 716-736,
enthält, oder eine Sequenz, die degeneriert ist oder die mit dieser eine Sequenzhomologie von mehr als 60 % hat oder die unter stark stringenten Bedingungen mit jeder der vorgenannten Sequenzen hybridisiert für die Herstellung einer Zusammensetzung zur KIV-Bekämpfung.

10. Expressions- oder Klonierungsvektor, umfassend ein Nucleinsäuremolekül, das eine Nucleotidsequenz, wie in Anspruch 5 definiert, umfaßt.

11. Wirtszelle oder transgener Organismus, enthaltend ein Nucleinsäuremolekül, das eine Nucleotidsequenz, wie in Anspruch 5 definiert, oder einen Expressionsvektor, wie in Anspruch 10 definiert, umfaßt.

12. Verfahren zur Herstellung eines synthetischen Polypeptids nach einem der Ansprüche 1 bis 3, umfassend Kultivieren von Wirtszellen, wie in Anspruch 11 definiert, unter Bedingungen, bei denen dieses Polypeptid exprimiert wird und Gewinnen dieses so hergestellten Polypeptids.

13. Verfahren zur Herstellung eines synthetischen Polypeptids nach einem der Ansprüche 1 bis 3, bei dem ein entsprechendes geschütztes oder immobilisiertes Polypeptid der Entfernung der Schutzgruppen oder Entfernung von einem festen Träger unterworfen wird.

14. Zusammensetzung zur KIV-Bekämpfung, umfassend ein oder mehrere synthetische Polypeptide nach einem der Ansprüche 1 bis 3 oder einen Expressionsvektor oder eine Wirtszelle, in die ein Nucleinsäuremolekül eingeführt ist, das eine Nucleotidsequenz nach Anspruch 5 umfaßt, zur Stimulierung einer Immunantwort, die gegen Polypeptide gerichtet ist, die durch das eingeführte Nucleinsäuremolekül codiert sind, zusammen mit einem oder mehreren pharmazeutischen Trägern oder Verdünnungsmitteln.

15. Zusammensetzung nach Anspruch 14, die zusätzlich ein oder mehrere andere antigene KIV-Proteine und/oder Polypeptide umfaßt.

16. Antikörper, die spezifisch an ein synthetisches Polypeptid nach einem der Ansprüche 1 bis 3 binden.

17. Verfahren zum Nachweisen von Antikörpern gegen KIV, wobei das Verfahren das Kontaktieren einer oder mehrere synthetischer Polypeptide nach einem der Ansprüche 1 bis 3 mit einer Probe, die diesen Antikörper umfaßt, und Nachweisen der Gegenwart eines zwischen diesem Polypeptid und dem Antikörper gebildeten Komplex, umfaßt.

18. Testkit zur Verwendung beim Nachweis von Antikörpern gegen KIV in einer Probe, umfassend ein oder mehrere synthetische Polypeptide nach einem der Ansprüche 1 bis 3 und Mittel zum Nachweisen der Komplexbildung zwischen diesem Polypeptid und dem Antikörper.

## Revendications

1. Polypeptide de synthèse comprenant une séquence aminoacide sélectionnée dans le groupe des séquences suivantes :
| Protéine d'enveloppe de FIV | | |
|---|---|---|
| SEQ ID NO | Numéro des résidus | Séquence |
| 1 | 377 à 417 | CQRTQSQPGLWLRAISSWKQRNRWEWRPDFKSKKMKISLQC ; et/ou |
| 2 | 462 à 490 | CRWNVGDNTSLIDTCGETQNVSGANPVDC ; et/ou |
| 3 | 354 à 377 | CGWWNQIAYYNSCRWESTDVKFHC ; et/ou |
| 5 | 417 à 445 | CNSTQNLTFAMRSSGDYGEVTGAWIEFGC ; et/ou |
| 6 | 320 à 339 | CTDPLQIPLINYTFGPNQTC ; et/ou |
| 7 | 161 à 190 | LFIGVGIYLGYAKAQVVWRLPPLVVPVEES ; et/ou |
| 8 | 248 à 267 | CRRGRIWKRWNETITGPSGC ; et/ou |
| 10 | 716 à 736 | NMTINQTIWNHGNITLGEWYN ; |
ou un fragment antigénique, un variant fonctionnellement équivalent conservant l'activité immunogénique ou un sel de ceux-ci acceptable du point de vue pharmaceutique.

2. Polypeptide de synthèse selon la revendication 1 pour l'utilisation dans la lutte contre le FIV.

3. Polypeptide de synthèse selon la revendication 1 ou la revendication 2, se présentant sous la forme d'une protéine de fusion comprenant un polypeptide supplémentaire fusionné à ladite séquence aminoacide, ou qui est couplé à un porteur protéinique ou polypeptidique.

4. Utilisation d'un polypeptide de synthèse selon l'une quelconque des revendications 1 à dans la préparation d'une composition pour lutter contre le FIV.

5. Molécule d'acide nucléique comprenant une séquence nucléotidique comprenant une région du gène env de FIV codant des résidus de la protéine d'enveloppe de FIV sélectionnés dans le groupe constitué par les résidus suivants :
(1) 377 à 417 ; et/ou
(2) 462 à 490 ; et/ou
(3) 354 à 377 ; et/ou
(5) 417 à 445 ; et/ou
(6) 320 à 339 ; et/ou
(7) 161 à 190 ; et/ou
(8) 248 à 267 ; et/ou
(10) 716 à 736 ;
ou une portion de celle-ci codant un fragment antigénique du FIV, ou une séquence qui est dégénérée ou a une homologie avec celle-ci supérieure à 60% ou qui s'hybride dans des conditions de stringence élevée avec l'une quelconque des séquences susmentionnées, pour l'utilisation dans la lutte contre le FIV.

6. Molécule d'acide nucléique comprenant une première séquence nucléotidique correspondant à une région du gène env de FIV codant des résidus de la protéine d'enveloppe de FIV sélectionnés dans le groupe constitué par les résidus suivants :
(1) 377 à 417 ; et/ou
(2) 462 à 490 ; et/ou
(3) 354 à 377 ; et/ou
(5) 417 à 445 ; et/ou
(6) 320 à 339 ; et/ou
(7) 161 à 190 ; et/ou
(8) 248 à 267 ; et/ou
(10) 716 à 736 ;
ou une portion de celle-ci codant un fragment antigénique de FIV, ou une séquence qui est dégénérée ou a une homologie avec celle-ci supérieure à 60% ou qui s'hybride dans des conditions de stringence élevée avec l'une quelconque des séquences susmentionnées, avec au moins une séquence nucléotidique supplémentaire flanquant ladite première séquence nucléotidique, la séquence nucléotidique supplémentaire ne comprenant pas plus de 900 bases.

7. Molécule d'acide nucléique selon la revendication 6 pour l'utilisation dans la lutte contre le FIV.

8. Utilisation d'une molécule d'acide nucléique selon la revendication 5 ou la revendication 6 dans la préparation d'une composition pour lutter contre le FIV.

9. Utilisation d'une molécule d'acide nucléique comprenant une séquence nucléotidique codant un polypeptide apte à diriger des anticorps neutralisants contre le FIV dont la séquence incorpore une ou plusieurs régions codant un déterminant antigénique des régions du gène env de FIV codant des résidus de la protéine d'enveloppe de FIV sélectionné dans le groupe de résidus suivants :
(1) 377 à 417 ; et/ou
(2) 462 à 490 ; et/ou
(3) 354 à 377 ; et/ou
(5) 417 à 445 ; et/ou
(6) 320 à 339 ; et/ou
(7) 161 à 190 ; et/ou
(8) 248 à 267 ; et/ou
(10) 716 à 736 ;
ou une séquence qui est dégénérée, a une homologie de séquence avec celle-ci supérieure à 60% ou qui s'hybride dans des conditions de stringence élevée avec l'une quelconque des séquences susmentionnées, pour la préparation d'une composition pour lutter contre le FIV.

10. Vecteur d'expression ou de clonage comprenant une molécule d'acide nucléique comprenant une séquence nucléotidique selon la revendication 5.

11. Cellule hôte ou organisme transgénique contenant une molécule d'acide nucléique comprenant une séquence nucléotidique selon la revendication 5 ou un vecteur d'expression selon la revendication 10.

12. Procédé de préparation d'un polypeptide de synthèse selon l'une quelconque des revendications 1 à 3, qui comprend la culture de cellules hôtes selon la revendication 11, dans des conditions dans lesquelles ledit polypeptide est exprimé, et la collecte dudit polypeptide ainsi produit.

13. Procédé pour la préparation de polypeptides de synthèse selon l'une quelconque des revendications 1 à 3 dans laquelle un polypeptide protégé ou immobilisé correspondant est soumis à la déprotection ou au retrait d'un support solide.

14. Composition pour lutter contre le FIV, comprenant un ou plusieurs polypeptides de synthèse selon l'une quelconque des revendications 1 à 3, ou un vecteur d'expression ou une cellule hôte dans lequel est insérée une molécule d'acide nucléique comprenant une séquence nucléotidique selon la revendication 5, pour la stimulation d'une réponse immune dirigée contre les polypeptides codés par la molécule d'acide nucléique insérée, avec un ou plusieurs porteurs ou diluants acceptables du point de vue pharmaceutique.

15. Composition selon la revendication 14, comprenant en outre une ou plusieurs protéines et/ou un ou plusieurs polypeptides de FIV antigéniques.

16. Anticorps se liant spécifiquement à un polypeptide de synthèse selon l'une quelconque des revendications 1 à 3.

17. Procédé pour détecter l'anticorps dirigé contre le FIV, ledit procédé comprenant la mise en contact d'un ou plusieurs polypeptides de synthèse selon l'une quelconque des revendications 1 à 3 avec un échantillon comprenant ledit anticorps, et la détection de la présence d'un complexe formé entre ledit polypeptide et ledit anticorps.

18. Trousse de test pour l'utilisation dans la détection d'anticorps dirigés contre le FIV dans un échantillon, comprenant un ou plusieurs polypeptides de synthèse selon l'une quelconque des revendications 1 à 3 et des moyens pour détecter la formation d'un complexe entre ledit polypeptide et ledit anticorps.
